# EUROPEAN PATENT SPECIFICATION

(11) **EP 2 173 364 B1**
(45) Date of publication and mention of the grant of the patent: **31.10.2018**
(21) Application number: 08826884.2
(22) Date of filing: 08.08.2008
(51) Int. Cl.: A61P 31/14, A61K 31/201, A61K 38/40, A61K 35/20

(54) **TREATING OR PREVENTING ROTAVIRUS INFECTION**
BEHANDLUNG ODER VERHINDERUNG VON ROTAVIRUSINFEKTIONEN
TRAITEMENT OU PRÉVENTION D'UNE INFECTION PAR UN ROTAVIRUS

(30) Priority: 09.08.2007 NZ 56052407
(43) Date of publication of application: 14.04.2010
(73) Proprietor: Fonterra Co-Operative Group Limited, Auckland 1010 (NZ)
(72) Inventor: MCCONNELL, Michelle Avril, Palmerston North (NZ); FITZPATRICK, Clare Elizabeth Ann, Palmerston North (NZ); MACGIBBON, Alastair Kenneth Hugh, Palmerston North (NZ)
(74) Representative: Walker, Ross Thomson
(86) International application number: PCT/NZ2008/000199
(87) International publication number: WO 2009/020405

(56) References cited:
- EP-A1- 1 350 519
- EP-A1- 1 623 717
- WO-A1-2005/107736
- WO-A1-2006/041316
- WO-A1-2007/055599
- DODGE J A ET AL: "Antiviral and antibacterial lipids in human milk and infant formula." ARCHIVES OF DISEASE IN CHILDHOOD FEB 1991 LNKD- PUBMED:2001121, vol. 66, no. 2, February 1991 (1991-02), pages 272-273, XP002605048 ISSN: 1468-2044
- OCHONICKY K ET AL: "Inhibitory activity of bovine milk fat globule membrane against sialic acid-dependent and -independent strains of rotavirus" JOURNAL OF DAIRY SCIENCE, vol. 88, no. Suppl. 1, 2005, page 365, XP009139959 & ANNUAL MEETING OF THE AMERICAN-DAIRY-SCIENCE-ASSOCIATION/AMERICA N-SOC IETY-OF-ANIMAL-SCIENCE/CANADIAN; CINCINNATI, OH, USA; JULY 24 -28, 2005 ISSN: 0022-0302
- KOOPERMAN, JS ET AL.: 'Infant formulas and gastrointestinal illness' AM J. PUBLIC HEALTH vol. 75, 1985, pages 477 - 480, XP008128737
- KOOPERMAN, JS ET AL.: 'Milk fat and gastrointestinal illness' AM J. PUBLIC HEALTH vol. 74, 1984, pages 1371 - 1373, XP008128736
- KVISTGAARD, AS ET AL.: 'Inhibitory effects of human and bovine milk constituents on rotavirus infections' J. DAIRY SCI. vol. 87, 2004, pages 4088 - 4096, XP055352613
- "Advanced Dairy Chemistry", 2006 vol. 2, Lipid, page 6,7,32,33,
- SMITH S ET AL: "Quantitative gas-liquid chromatographic analysis of rodent milk triglycerides.", JOURNAL OF LIPID RESEARCH JAN 1968, vol. 9, no. 1, January 1968 (1968-01), pages 52-57, ISSN: 0022-2275

## Description

### FIELD OF THE INVENTION

The present invention relates to use of one or more of conjugated linoleic acid (CLA), high CLA milk fat, or one or more bovine milk lipid compositions to treat or prevent rotavirus infection.

### BACKGROUND

Milk is a rich biological fluid that provides an important source of nutrition in the neonate. In addition, it contains agents for immune system development, function, and support that are necessary for infant development.

Rotaviruses are Group III (dsRNA) viruses of the family Reoviridae and account for approximately 138 million cases of infantile diarrhea per year. 95% of children will experience an episode of rotavirus diarrhea by the time they turn five. Worldwide, rotavirus infection is the most common cause of hospitalisation for diarrhea (Parashar et al 2003.). An estimated 1,205 children die from rotavirus disease each day. One in five children will visit the doctor with rotavirus, one child in 65 will require hospitalization, and approximately one child in 293 will die. Most deaths occur in the Indian subcontinent, sub-Saharan Africa and South America (Parashar et al 2003).

Rotavirus particles are formed of three concentric layers of proteins. Two proteins in the outer layer of the virus, VP4 and VP7, are implicated in the initial interaction of the virus with the host cell. VP4 forms spikes that extend from.the surface of the viral particle and has been identified as the viral attachment polypeptide while VP7 is a calcium-binding glycoprotein that forms the smooth surface of the virion and is thought to be involved in the post attachment process (Mendez et al 1999, Isa et al 2006). Entry into the cell is a complex process involving interactions between outer layer proteins of the virus and cell surface molecules. *In vivo,* rotaviruses infect via the mature enterocytes of the villi of the small intestine whereas *in vitro* rotaviruses bind to a variety of cell lines but only some of these become infected. Isa et al (2006) reports that initial binding of a virus to the cell surface is promiscuous and that the subsequent interaction of the virus with specific post-attachment receptors is responsible for the entry of the virus into the cell.

Rotaviruses are classified into two types based on their ability to infect cells that have been treated with neuraminidase to remove the sialic acid (N-acetylneuraminic acid) residues on the host cells. Rotaviruses from some animals need sialic residues on the cell surface in order to attach whereas many others including human rotavirus strains do not. These are described as neuraminidase sensitive (or sialic acid dependent) and neuraminidase resistant (or sialic acid independent) strains respectively (Isa et al, 2006). Sialic acid derivatives have been reported to inhibit viral binding of cells susceptible to animal rotaviruses, but not human rotaviruses (Guo et al, 1999). Ganglioside GM1a on the host cell surface has been reported to play a role as a receptor in human rotavirus infection (Guo et al, 1999). Neuraminidase resistant strains are reported to recognize gangliosides with internal sialic acids, which are resistant to neuraminidase treatment (Delorme et al, 2001), in contrast to neuraminidase sensitive viruses which bind to external residues in gangliosides (Isa et al, 2006).

A number of compounds (theaflavins, protease inhibitors, egg yolk immunoglobulin, synthetic sulphated sialyl lipid NMSO₃, human milk lactadherin, MUC1 mucin and bovine macromolecular whey protein (MMWP) fraction) are reported to suppress rotavirus infection in vitro and *in vivo* but none has been successfully used clinically (Bojsen et al, 2007; Peterson et al, 2001; Takahashi et al, 2002; Isa et al, 2006). Much of this work was done with animal strains of rotavirus rather than human strains e.gi MUC1 mucin and bovine macromolecular whey protein fraction (Bojsen et al 2007). A study was carried out using a whey protein concentrate produced by Fonterra Cooperative Group limited that showed a reduction in rotaviral shedding in a mouse model using a mouse strain of rotavirus EDIM (Wolber et al, 2005). Kvistgaard et al, 2004, reported that bovine lactoferrin, bovine lactadherin and bovine MUC1 mucin could not prevent human Wa rotavirus infection in vitro.

The normal treatment for rotavirus diarrhea has been rehydration therapy, but this therapy treats the symptoms, not the cause of the infection. Anti-diarrheal medication is not generally recommended for use by children. (Yung et al, 2005).

WO2006/041316 discloses a process for producing dairy products having lower levels of neutral lipids, and/or higher levels of polar lipids, by extraction using near critical carbon dioxide or dimethyl ether.

Ochonicky K et al in the Journal of Dairy Science, vol. 88, no. Suppl. 1, 2005, page 365, Annual Meeting of the American-Dairy-Science-Associtiion/American-Society-of-animal-Science/Canadian; Cincinnati, HO, USA; July 24-28, 2005 ISSN; 0022-0302 relates to "Inhibitory activity of bovine milk fat globule membrane against sialic acid-dependent and - independent strains of rotavirus".

Therefore, the need exists for alternative therapies to treat or prevent rotavirus infection.

### SUMMARY OF THE INVENTION

In a first aspect of the present invention there is provided an agent comprising
(a) one or more bovine milk fat compositions comprising about 5 to about 95% w/w lipid, about 0 to about 75% w/w protein, about 5 to about 85% w/w phospholipid and about 0 to about 5% w/w ganglioside selected from
   (i) one or more phospholipid-enriched fractions of milk fat,
   (ii) one or more ganglioside-enriched fractions of milk fat,
   (iii) one or more hydrolysates of any one or more of (i) to (ii), and
   (iv) a combination of any two or more of (i) to (iii);
   for the use in treating or preventing rotavirus infection in a subject, wherein the rotavirus is a neuraminidase-resistant rotavirus.

The following embodiments may relate to any of the above aspects.

In one embodiment the rotavirus is a human rotavirus, that is, a rotavirus strain that infects humans. In another embodiment the rotavirus is a non-human rotavirus strain, such as an avian (such as a chicken, pigeon, or turkey rotavirus strain), bovine, canine, caprine, equine, feline, leporine, murine, ovine, porcine, or simian rotavirus strain. In one embodiment the rotavirus is a neuraminidase-sensitive rotavirus. In another embodiment the rotavirus is a neuraminidase-resistant rotavirus. In yet another embodiment the rotavirus is a neuraminidase-resistant human rotavirus. In still another embodiment the neuraminidase-resistant human rotavirus is the *Wa* human rotavirus strain. In one embodiment the subject is a human. In one embodiment the subject is an adult, a child, or an infant, or an immunocompromised adult, child, or infant. In another embodiment the subject is a child, an infant, an immunocompromised child, or an immunocompromised infant. In another embodiment the subject is an adult, an adult over the age of 55, an immunocompromised adult, or an immunocompromised adult over the age of 55.

In one embodiment the agent treats or prevents rotavirus infection by reducing viral adhesion to cells. In another embodiment the agent treats or prevents rotavirus infection by reducing viral entry into cells. In another embodiment the agent treats or prevents rotavirus infection by reducing viral replication in cells. In another embodiment the agent treats or prevents rotavirus infection by reducing viral packaging in cells. In another embodiment the agent treats or prevents rotavirus infection by reducing viral cell lysis. In one embodiment the agent treats or prevents diarrheagenic rotavirus infection. In another embodiment the agent treats or prevents diarrhea caused by rotavirus infection.

In one embodiment the ganglioside-enriched fraction comprises GD3 or GM3 or a combination thereof.

In one embodiment the phospholipid-enriched fraction comprises one or more phosphatidylethanolamines, one or more phosphatidylinositols, one or more phosphatidylserines, one or more phosphatidylcholines, one or more sphingolipids (including one or more sphingomyelins, one or more dihydrosphingomyelins, one or more ceramides, one or more cerebrosides, or one or more gangliosides, or any combination of any two or more thereof), one or more lysophospholipids (phospholipids with one fatty acid lost), or any combination of any two or more thereof.

In one embodiment the agent comprises one or more glycerides (including one or more monoglycerides, one or more diglycerides, or one or more triglycerides, or any combination of any two or more thereof), one or more phospholipids (including one or more phosphatidylethanolamines, one or more phosphatidylinositols, one or more phosphatidylserines, one or more phosphatidylcholines, or one or more sphingolipids (as described above), or any combination of any two or more thereof), one or more lysophospholipids (phospholipids with one fatty acid lost), one or more ceramides, one or more ether glycerophospholipids, one or more cerebrosides (including one or more glucosylceramides or one or more lactosylceramides, or combinations thereof), one or more sulfatides, or one or more gangliosides (including one or more monosialogangliosides, one or more disialoganglioside, or one or more polysialogangliosides, or combinations thereof), or any combination of any two or more thereof.

In one embodiment the agent is administered as a component of a lipid composition. Preferred lipid compositions include animal, plant and marine oils and fats and lipids produced by fermentation with microorganisms. Preferred animal fats include but are not limited to dairy fats, particularly bovine milk fat, including cream.

In one embodiment the agent is selected from cream, butter, ghee, anhydrous milk fat (AMF) (typically produced by phase inversion of cream or dehydration of butter), buttermilk, butter serum, beta serum, hard milk fat fractions from one or more stages of fractionation (including H, SH, and SSH fractions), soft milk fat fractions from one or more stages of fractionation (including S, SS, and SSS fractions), combinations of hard milk fat fractions, combinations of soft milk fat fractions, combinations of hard milk fat fractions and soft milk fat fractions, sphingolipid fractions, milk fat globule (or "globular) membrane lipid-enriched fractions (including, for example, sphingolipids, ceramides, and cerebrosides), phospholipid fractions, and complex lipid fractions, CLA-enriched milk fat, CLA-enriched milk fat fractions, and any combinations of any two or more thereof, and hydrolysates thereof, and fractions of the hydrolysates, and combinations of hydrolysed and/or non-hydrolysed fractions. These fractions may be obtained from whole milk or colostrum, and any derivatives of whole milk or colostrum, including cream, cultured cream, and whey cream (milk lipid obtained from whey, including acid whey or cheese whey, preferably cheese whey). Cultured cream is cream from whole milk or colostrum that has been fermented with acid-producing microorganisms, preferably lactic acid bacteria.

In one embodiment the milk fat or fraction thereof is selected from cream, butter, ghee, anhydrous milk fat (AMF) (typically produced by phase inversion of cream or dehydration of butter), buttermilk, butter serum, beta serum, hard milk fat fractions from one or more stages of fractionation (including H, SH, and SSH fractions), soft milk fat fractions from one or more stages of fractionation (including S, SS, and SSS fractions), combinations of hard milk fat fractions, combinations of soft milk fat fractions, combinations of hard milk fat fractions and soft milk fat fractions, and any combination of any two or more thereof.

In one embodiment the AMF fraction is selected from one or more hard milk fat fractions (such as H, SH, and SSH fractions), one or more soft milk fat fractions (such as S, SS, and SSS fractions), combinations of hard milk fat fractions, combinations of soft milk fat fractions, combinations of hard milk fat fractions and soft milk fat fractions, and any combination of any two or more thereof.

In one embodiment the phospholipid-enriched fraction is selected from buttermilk, one or more buttermilk fractions, butter serum, one or more butter serum fractions, beta serum, one or more beta serum fractions, one or more sphingolipid fractions, one or more milk fat globule membrane lipid fractions, one or more phospholipid fractions, one or more complex lipid fractions, and any combination of any two or more thereof.

In one embodiment the ganglioside-enriched fraction is selected from buttermilk, one or more buttermilk fractions, butter serum, one or more butter serum fractions, beta serum, one or more beta serum fractions, one or more GD3-enriched fractions of beta serum, one or more GM3-enriched fractions of beta serum, one or more GD3- and GM3-enriched fractions of beta serum, and any combination of any two or more thereof.

In some embodiments the fraction comprises
(a) about 5 to about 100% w/w lipid, or
(b) about 40 to about 100% w/w lipid, or
(c) about 5 to about 95% w/w lipid and about 0 to about 75% w/w protein, or
(d) about 15 to about 95% w/w lipid and about 0 to about 75% w/w protein, or
(e) about 5 to about 95% w/w lipid, about 0 to about 75% w/w protein, about 5 to about 85% w/w phospholipids and about 0 to about 5% w/w ganglioside, or
(f) about 15 to about 95% w/w lipid, about 0 to about 65% w/w protein, about 5 to about 70% w/w phospholipids and about 0 to about 2.5% w/w ganglioside.

In some embodiments the fraction comprises at least about 5, 10, 15, 20, 25, 30, 35, 40, 45, 50, 55, 60, 65, 70, 75, 80, 85, 90 or 95% w/w phospholipid, and useful ranges may be selected between any of these values (for example, about 5 to about 95%, about 10 to about 95%, about 15 to about 95%, about 20 to about 95%, about 25 to about 95%, about 30 to about 95%, about 35 to about 95%, about 40 to about 95%, about 45 to about 95%, about 50 to about 95%, about 10 to about 70%, about 15 to about 70%, about 20 to about 70%, about 25 to about 70%, about 30 to about 70%, about 35 to about 70%, about 40 to about 70%, about 45 to about 70%, and about 50 to about 70% w/w phospholipid).

In some embodiments the fraction comprises at least about 0.1, 0.5, 1, 2, 3, 4, 5, 6, 7, 8, 9, 10, 11, 12, 13, 14, 15, 16, 17, 18, 19, 20, 21, 22, 23, 24, 25, 26, 27, 28, 29 or 30% w/w of one or more phospholipids selected independently from phosphatidylcholine, phosphatidylethanolamine, sphingomyelin, phosphatidylserine, and phosphatidylinositol, and useful ranges may be selected between any of these values (for example, about 0.1 to about 30%, about 0.5 to about 30%, about 1 to about 30%, about 2 to about 30%, about 3 to about 30%, about 4 to about 30%, about 5 to about 30%, about 10 to about 30%, about 15 to about 30%, about 20 to about 30%, about 0.1 to about 5%, about 0.5 to about 5%, about 1 to about 5%, about 2 to about 5%, about 3 to about 5%, about 0.1 to about 10%, about 0.5 to about 10%, about 1 to about 10%, about 2 to about 10%, about 3 to about 10%, about 4 to about 10%, about 5 to about 10%, about 6 to about 10%, about 0.1 to about 20%, about 0.5 to about 20%, about 1 to about 20%, about 2 to about 20%, about 3 to about 20%, about 4 to about 20%, about 5 to about 20%, about 10 to about 20%, about 15 to about 20% w/w of one or more phospholipids selected independently from phosphatidylcholine, phosphatidylethanolamine, sphingomyelin, phosphatidylserine, and phosphatidylinositol).

In some embodiments the fraction comprises
(a) about 25 to about 35% w/w protein, about 15 to about 25% w/w lipid, and about 5 to about 12% w/w phospholipid, or
(b) about 25 to about 35% w/w protein, about 15 to about 25% w/w lipid, about 5 to about 12% w/w phospholipid, about 5 to about 10% w/w MFGM protein, and about 0.2 to about 0.9% w/w ganglioside, or
(c) about 25 to about 35% w/w protein, about 15 to about 25% w/w lipid, about 5 to about 12% w/w phospholipid, about 1 to about 5% w/w phosphatidylcholine, about 1.5 to about 6% w/w phosphatidylethanolamine, about 1 to about 5% w/w sphingomyelin, about 0.5 to about 2% w/w phosphatidylserine, about 0.1 to 2% w/w phosphatidylinositol, about 5 to about 10% w/w MFGM protein, and about 0.2 to about 0.9% w/w ganglioside, or
(d) about 40 to about 60% w/w protein, about 25 to about 45% w/w lipid, and about 10 to about 25% w/w phospholipid, or
(e) about 40 to about 60% w/w protein, about 25 to about 45% w/w lipid, about 10 to about 25% w/w phospholipid, about 5 to about 20% w/w MFGM protein, and about 0.5 to about 2.0% w/w ganglioside, or
(f) about 46 to about 52% w/w protein, about 28 to about 40% w/w lipid, about 11 to about 16% w/w phospholipid, about 2 to about 6% w/w phosphatidylcholine, about 3 to about 8% w/w phosphatidylethanolamine, about 2.5 to about 7% w/w sphingomyelin, about 0.5 to about 3% w/w phosphatidylserine, about 0.5 to 2% w/w phosphatidylinositol, about 5 to about 15% w/w MFGM protein, and about 0.5 to about 0.9% w/w ganglioside, or
(g) about 50 to about 70% w/w protein, about 12 to about 32% w/w lipid, and about 5 to about 25% w/w phospholipid, or
(h) about 50 to about 70% w/w protein, about 12 to about 32% w/w lipid, about 5 to about 25% w/w phospholipid, about 2 to about 8 % w/w phosphatidylcholine, about 2 to about 10% w/w phosphatidylethanolamine, about 2 to about 8% w/w sphingomyelin, and about 1 to about 3% w/w phosphatidylserine, about 10 to about 20% w/w MFGM protein, and about 0.5 to about 2.5% w/w ganglioside, or
(i) about 56 to about 65% w/w protein, about 18 to about 28 % w/w lipid, about 8 to about 20% w/w phospholipid, about 2 to about 8 % w/w phosphatidylcholine, about 2 to about 10% w/w phosphatidylethanolamine, about 2 to about 8% w/w sphingomyelin, and about 1 to about 3% w/w phosphatidylserine, and about 0.5 to 3% w/w phosphatidylinositol, about 10 to about 20% w/w MFGM protein, and about 0.5 to about 2.5% w/w ganglioside, or
(j) about 0 to about 10% w/w protein, about 85 to about 97% w/w lipid, and about 25 to about 35% w/w phospholipid, or
(k) about 0 to about 10% w/w protein, about 85 to about 97% w/w lipid, about 25 to about 35% w/w phospholipid, about 5 to about 10 % w/w phosphatidylcholine, about 7 to about 13% w/w phosphatidylethanolamine, about 4 to about 9% w/w sphingomyelin, about 2 to about 5% w/w phosphatidylserine, about 1 to about 3% w/w phosphatidylinositol, about 0 to about 5 % w/w MFGM protein, and about 1 to about 3% w/w gangliosides, or
(l) about 10 to about 15% w/w protein, about 80 to about 95% w/w lipid, and about 60 to about 80% w/w phospholipid, or
(m) about 10 to about 15% w/w protein, about 80 to about 95% w/w lipid, about 60 to about 80% w/w phospholipid, about 10 to about 20 % w/w phosphatidylcholine, about 18 to about 28% w/w phosphatidylethanolamine, about 10 to about 20% w/w sphingomyelin, about 4 to about 12% w/w phosphatidylserine, about 2 to about 10% w/w phosphatidylinositol, about 0 to about 5% w/w MFGM protein, and about 1 to about 5% w/w gangliosides, or
(n) about 75 to about 99% w/w lipid and about 15 to 35% w/w phospholipid, or
(o) about 80 to about 90% w/w lipid, about 5 to about 15% w/w phosphatidylcholine, about 5 to about 15% w/w phosphatidylethanolamine, about 4 to about 10% w/w sphingomyelin, and about 0.1 to about 2% w/w phosphatidylserine, or
(p) about 80 to about 90% w/w lipid, about 20 to 30% w/w phospholipid, about 5 to about 15% w/w phosphatidylcholine, about 5 to about 15% w/w phosphatidylethanolamine, about 5 to about 10% w/w sphingomyelin, about 0.5 to about 1.5% w/w phosphatidylserine, and about 0.1 to about 1.2% w/w phosphatidylinositol, or
(q) about 75 to about 95% w/w lipid and about 50 to about 90% w/w phospholipid, or
(r) about 80 to about 90% w/w lipid, about 10 to about 30% w/w phosphatidylcholine, about 12-to about 22% w/w phosphatidylethanolamine, about 12 to about 22% w/w sphingomyelin, and about 1 to about 3% w/w phosphatidylserine, or
(s) about 75 to about 95% w/w lipid, about 50 to about 90% w/w phospholipid, about 10 to about 45% w/w phosphatidylcholine, about 12 to about 25% w/w phosphatidylethanolamine, about 12 to about 25% w/w sphingomyelin, about 1 to about 6% w/w phosphatidylserine, and about 0.5 to 4% w/w phosphatidylinositol, or
(t) about 80 to about 90% w/w lipid, about 65 to about 75% w/w phospholipid, about 10 to about 30% w/w phosphatidylcholine, about 12 to about 22% w/w phosphatidylethanolamine, about 12 to about 22% w/w sphingomyelin, about 1 to about 3% w/w phosphatidylserine, and about 0.5 to 3% w/w phosphatidylinositol, or
(u) about 25 to about 45% w/w lipid and about 0.2 to about 1% w/w ganglioside GD3, or
(v) about 25 to about 45% w/w lipid, about 10 to about 30% w/w phospholipids, and about 0.2 to about 1% w/w ganglioside, or
(w) about 25 to about 45% w/w lipid, about 10 to about 30% w/w phospholipids, about 2 to about 5% w/w phosphatidylcholine, about 3 to about 7% w/w phosphatidylethanolamine, about 2 to about 5% w/w sphingomyelin, about 2 to about 12% w/w phosphatidylserine, about 1 to about 5% w/w phosphatidylinositol, and about 0.2 to about 1% w/w ganglioside, or
(x) about 20 to about 40% w/w lipid and about 0.8 to about 2% w/w ganglioside GD3, or
(y) about 20 to about 40% w/w lipid, about 5 to about 30% w/w phospholipids, and about 0.8 to about 3.5% w/w ganglioside, or
(z) about 20 to about 40% w/w lipid, about 5 to about 30% w/w phospholipids, about 1 to about 5% w/w phosphatidylcholine, about 2 to about 8% w/w phosphatidylethanolamine, about 0.5 to about 5% w/w sphingomyelin, about 1 to about 10% w/w phosphatidylserine, about 1 to about 6% w/w phosphatidylinositol, and about 0.8 to about 3.5% w/w ganglioside.

In one embodiment, the fraction comprises at least about 70% total lipids, at least about 12% phosphatidylcholine, at least about 6% phosphatidylethanolamine, at least about 6% sphingomyelin, and at least about 1% phosphatidylserine. In a preferred fraction of this embodiment, the fraction comprises about 1.8% butyric acid (4:0), about 0.3% capric acid (10:0), about 0.5% lauric acid (12:0), about 7.4% myristic acid (14:0), about 14.1% myristoleic acid (14:1), about 1.0% pentadecanoic acid (15:0), about 26.0% palmitic acid (16:0), about 1.7% palmitoleic acid (16:1), about 0.6% margaric acid (17:0), about 0.3% heptadecenoic acid (17:1), about 11.9% stearic acid (18:0), about 39.0% oleic acid (18:1), about 5.0% linoleic acid (18:2), about 2.0% linolenic acid (18:3), about 0.3% arachidic acid (20:0), and about 0.8% cholesterol. In a preferred embodiment, the fatty acid composition of this fraction is substantially as described for the Phospholipid Concentrate PC500™ phospholipid fraction in the examples below. Alternatively, the fraction is a hydrolysate of the fraction of this embodiment.

In another embodiment, the fraction comprises at least about 80% total lipids, at least about 30% phosphatidylcholine, at least about 6% phosphatidylethanolamine, at least about 15% sphingomyelin, and at least about 2% phosphatidylserine. In a preferred fraction of this embodiment, the fraction comprises about 6.6% myristic acid (14:0), about 27.0% palmitic acid (16:0), about 1.3% palmitoleic acid (16:1), about 2.3% margaric acid (17:0), about 14% stearic acid (18:0), about 38.0% oleic acid (18:1), about 6.5% linoleic acid (18:2), about 2.0% linoleic (18:3), and about 0.1% cholesterol. In a preferred embodiment, the fatty acid composition of this fraction is substantially as described for the Phospholipid Concentrate PC600™ or PC700™ phospholipid fractions in the examples below. Alternatively, the fraction is a hydrolysate of the fraction of this embodiment.

In one embodiment, the fraction comprises at least about 30% total lipids, at least about 0.5% ganglioside GD3, and at least about 0.4% ganglioside GM3. In a preferred embodiment, the fatty acid composition of this fraction is substantially as described for the G500™ ganglioside fraction in the examples below. Alternatively, the fraction is a hydrolysate.

In another embodiment, the fraction comprises at least about 30% total lipids, at least about 1.2% ganglioside GD3, and at least about 0.2% ganglioside GM3. In a preferred embodiment, the fatty acid composition of this fraction is substantially as described for the Ganglioside G600™ ganglioside fraction in the examples below. Alternatively, the fraction is a hydrolysate of the fraction of this embodiment.

In one embodiment a composition useful herein comprises, consists essentially of, or consists of at least about 0.1, 0.2, 0.5, 1, 5, 10, 15, 20, 25, 30, 35, 40, 45, 50, 55, 60, 65, 70, 75, 80, 85, 90, 95, 99, 99.5, 99.8 or 99.9% by weight of one or more agents described above and useful ranges may be selected between any of these foregoing values (for example, from about 0.1 to about 50%, from about . 0.2 to about 50%, from about 0.5 to about 50%, from about 1 to about 50%, from about 5 to about 50%, from about 10 to about 50%, from about 15 to about 50%, from about 20 to about 50%, from about 25 to about 50%, from about 30 to about 50%, from about 35 to about 50%, from about 40 to about 50%, from about 45 to about 50%, from about 0.1 to about 60%, from about 0.2 to about 60%, from about 0.5 to about 60%, from about 1 to about 60%, from about 5 to about 60%, from about 10 to about 60%, from about 15 to about 60%, from about 20 to about 60%, from about 25 to about 60%, from about 30 to about 60%, from about 35 to about 60%, from about 40 to about 60%, from about 45 to about 60%, from about 0.1 to about 70%, from about 0.2 to about 70%, from about 0.5 to about 70%, from about 1 to about 70%, from about 5 to about 70%, from about 10 to about 70%, from about 15 to about 70%, from about 20 to about 70%, from about 25 to about 70%, from about 30 to about 70%, from about 35 to about 70%, from about 40 to about 70%, from about 45 to about 70%, from about 0.1 to about 80%, from about 0.2 to about 80%, from about 0.5 to about 80%, from about 1 to about 80%, from about 5 to about 80%, from about 10 to about 80%, from about 15 to about 80%, from about 20 to about 80%, from about 25 to about 80%, from about 30 to about 80%, from about 35 to about 80%, from about 40 to about 80%, from about 45 to about 80%, from about 0.1 to about 90%, from about 0.2 to about 90%, from about 0.5 to about 90%, from about 1 to about 90%, from about 5 to about 90%, from about 10 to about 90%, from about 15 to about 90%, from about 20 to about 90%, from about 25 to about 90%, from about 30 to about 90%, from about 35 to about 90%, from about 40 to about 90%, from about 45 to about 90%, from about 0.1 to about 99%, from about 0.2 to about 99%, from about 0.5 to about 99%, from about 1 to about 99%, from about 5 to about 99%, from about 10 to about 99%, from about 15 to about 99%, from about 20 to about 99%, from about 25 to about 99%, from about 30 to about 99%, from about 35 to about 99%, from about 40 to about 99%, and from about 45 to about 99%).

In one embodiment a composition useful herein comprises, consists essentially of, or consists of at least about 0.001, 0.01, 0.05, 0.1, 0.15, 0.2, 0.3, 0.4, 0.5, 1, 2, 3, 4, 5, 6, 7, 8, 9, 10, 11, 12, 13, 14, 15, 16, 17, 18 or 19 grams of one or more agents described above and useful ranges may be selected between any of these foregoing values (for example, from about 0.01 to about 1 grams, about 0.01 to about 10 grams, about 0.01 to about 19 grams, from about 0.1 to about 1 grams, about 0.1 to about 10 grams, about 0.1 to about 19 grams, from about 1 to about 5 grams, about 1 to about 10 grams, about 1 to about 19 grams, about 5 to about 10 grams, and about 5 to about 19 grams).

In one embodiment a composition useful herein comprises, consists essentially of, or consists of about 0.1, 0.5, 1, 5, 10, 15, 20, 25, 30, 35, 40, 45, 50, 55, 60, 65, 70, 75, 80, 85, 90, 95, 97, 99, or 99.9 % by weight of fresh whole milk or a milk derivative and useful ranges may be selected between any of these foregoing values (for example, from about 0.1 to about 50%, from about 0.2 to about 50%, from about 0.5 to about 50%, from about 1 to about 50%, from about 5 to about 50%, from about 10 to about 50%, from about 15 to about 50%, from about 20 to about 50%, from about 25 to about 50%, from about 30 to about 50%, from about 35 to about 50%, from about 40 to about 50%, and from about 45 to about 50%). The milk derivative is preferably selected from recombined, powdered or fresh skim milk, recombined or reconstituted whole or skim milk powder, skim milk concentrate, skim milk retentate, concentrated milk, ultrafiltered milk retentate, milk protein concentrate (MPC), milk protein isolate (MPI), calcium depleted milk protein concentrate (MPC), low fat milk, low fat milk protein concentrate (MPC), casein, caseinate, milk fat, cream, butter, ghee, anhydrous milk fat (AMF), buttermilk, butter serum, beta serum, hard milk fat fractions, soft milk fat fractions, sphingolipid fractions, milk fat globular membrane fractions, milk fat globular membrane lipid fractions, phospholipid fractions, complex lipid fractions, colostrum, a colostrum fraction, colostrum protein concentrate (CPC), colostrum whey, an immunoglobulin fraction from colostrum, whey (including sweet whey, lactic acid whey, mineral acid whey, or reconstituted whey powder), whey protein isolate (WPI), whey protein concentrate (WPC), a composition derived from any milk or colostrum processing stream, a composition derived from the retentate or permeate obtained by ultrafiltration or microfiltration of any milk or colostrum processing stream, a composition derived from the breakthrough or adsorbed fraction obtained by chromatographic (including but not limited to ion and gel permeation chromatography) separation of any milk or colostrum processing stream, extracts of any of these milk derivatives including extracts prepared by multistage fractionation, differential crystallisation, solvent fractionation, supercritical fractionation, near critical fractionation, distillation, centrifugal fractionation, or fractionation with a modifier (e.g. soaps or emulsifiers), hydrolysates of any of these derivatives, fractions of the hydrolysates, and any combination of any two or more of these derivatives, including combinations of hydrolysed and/or non-hydrolysed fractions. It should be understood that the source of these derivatives may be milk or colostrum or a combination thereof.

In one embodiment a composition useful herein further comprises a pharmaceutically acceptable carrier. In another embodiment the composition is or is formulated as a food, drink, food additive, drink additive, dietary supplement, nutritional product, medical food, enteral feeding product, parenteral feeding product, meal replacement, cosmeceutical, nutraceutical, medicament, or pharmaceutical. In one embodiment the composition is in the form of a tablet, a caplet, a pill, a hard or soft capsule or a lozenge. In one embodiment the composition is in the form of a cachet, a powder, a dispensable powder, granules, a suspension, an elixir, a liquid, or any other form that can be added to food or drink, including for example water, milk or fruit juice. In one embodiment the composition further comprises one or more constituents (such as antioxidants) which prevent or reduce degradation of the composition during storage or after administration. These compositions may include any edible consumer product which is able to carry lipid. Examples of suitable edible consumer products include aqueous products, baked goods, confectionary products including chocolate, gels, ice creams, reconstituted fruit products, snack bars, food bars, muesli bars, spreads, sauces, dips, dairy products including yoghurts and cheeses, drinks including dairy and non-dairy based drinks, milk, milk powders, sports supplements including dairy and non-dairy based sports supplements, fruit juice, food additives such as protein sprinkles, dietary supplement products including daily supplement tablets, weaning foods and yoghurts, and formulas such as infant formula, follow-on formula, or growing-up formula, in powder or liquid form. Suitable nutraceutical compositions useful herein may be provided in similar forms.

In one embodiment the composition may further comprise or the agent may be in combination with an anti-viral agent such as one or more anti-viral agents selected from theaflavins, protease inhibitors, egg yolk immunoglobulin, synthetic sulphated sialyl lipid NMSO₃, human milk lactadherin (a glycoprotein of the milk fat globule membrane), MUC1 mucin, bovine macromolecular whey protein fraction, or combinations thereof. In one embodiment, a composition useful herein includes or is administered simultaneously or sequentially with milk components such as whey protein, whey protein fractions (including acidic or basic whey protein fractions or a combination thereof), glycomacropeptide, lactoferrin, iron-lactoferrin, a functional lactoferrin or iron-lactoferrin variant, a function lactoferrin or iron-lactoferrin fragment, a vitamin D, or calcium, or any combination of any two or more thereof. Preferably the composition may further comprise or the agent may be in combination with lactoferrin, iron-lactoferrin, a functional lactoferrin or iron-lactoferrin variant, a function lactoferrin or iron-lactoferrin fragment, or a combination of any two or more thereof.

It is intended that reference to a range of numbers disclosed herein (for example, 1 to 10) also incorporates reference to all rational numbers within that range (for example, 1, 1.1, 2, 3, 3.9, 4, 5, 6, 6.5, 7, 8, 9 and 10) and also any range of rational numbers within that range (for example, 2 to 8, 1.5 to 5.5 and 3.1 to 4.7) and, therefore, all sub-ranges of all ranges expressly disclosed herein are hereby expressly disclosed. These are only examples of what is specifically intended and all possible combinations of numerical values between the lowest value and the highest value enumerated are to be considered to be expressly stated in this application in a similar manner.

In this specification where reference has been made to patent specifications, other external documents, or other sources of information, this is generally for the purpose of providing a context for discussing the features of the invention. Unless specifically stated otherwise, reference to such external documents is not to be construed as an admission that such documents, or such sources of information, in any jurisdiction, are prior art, or form part of the common general knowledge in the art.

### BRIEF DESCRIPTION OF THE DRAWINGS

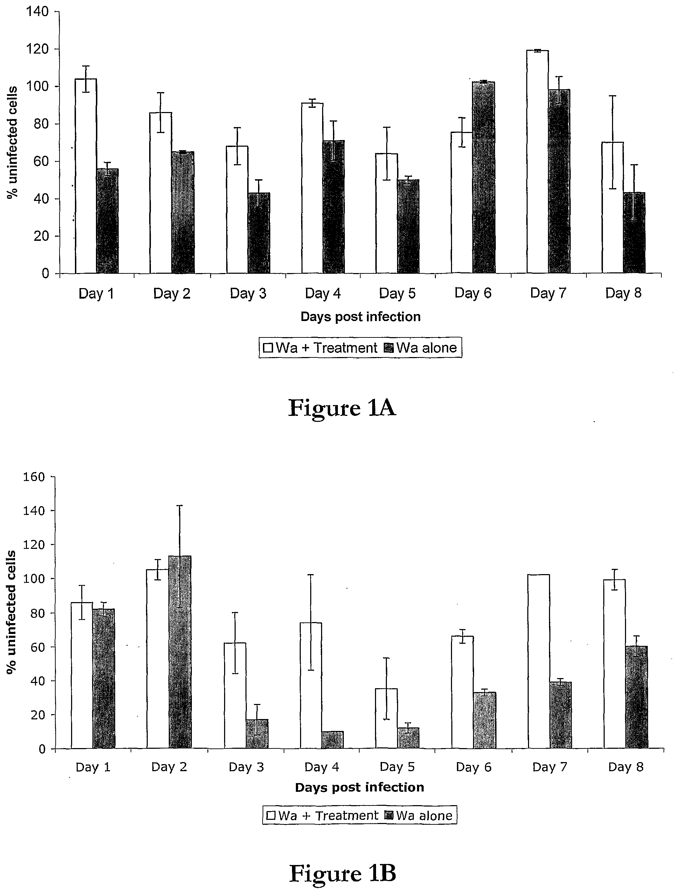

Figure 1 two graphs showing the percentage of uninfected cells in animals treated according to Example 2 with (A) high CLA milk fat or (B) a G600™ ganglioside fraction of milk fat from Fonterra Co-operative Group Limited.

### DETAILED DESCRIPTION OF THE INVENTION

The examples below demonstrate prevention of rotavirus cell adhesion *in vitro* by various milk fat fractions and the ability of milk fat fractions to treat or prevent diarrhea in an animal model infected with the *Wa* human rotavirus strain.

### 1. Definitions

The terms "anhydrous milk fat" and "AMF" are used interchangeably herein and refer to the milk fat fraction produced by the almost complete removal of water and non-fat material by phase inversion of cream or dehydration of butter. AMF (also known as "anhydrous butteroil" if additives are present) is typically prepared from cream or butter from whole milk but may also be prepared from colostrum. Methods commonly used for the preparation of AMF are disclosed in Bylund (Ed., 1995), incorporated herein in its entirety. Preferred AMF is typically about 60%, about 70%, about 80%, about 90%, about 95%, greater than about 95%, about 96%, about 97%, about 98%, about 99%, about 99.5%, or 100% lipid, with AMF of about 98% to about 100%, particularly about 99% lipid, 99.5% lipid or greater being more preferred. Food regulations commonly require <0.2% moisture for AMF or anhydrous butteroil and <0.7% moisture for butteroil. AMF is frequently further fractionated into "hard" (H) and "soft"(S) fractions, the latter can be further fractionated into "soft hard" (SH) and "soft soft" (SS) fractions, the latter can again be further fractionated into "soft soft hard" (SSH) and "soft soft soft" (SSS) fractions. As will be appreciated, each fraction differs in fatty acid composition. Nonlimiting exemplary fatty acid compositions for AMF and derivative fractions are shown in Tables 1 to 5 below.

**Table 1: Exemplary AMF composition**

| **Fatty acid component** | **Mean (% w/w)** | **Min (% w/w)** | **Max (% w/w)** |
|---|---|---|---|
| c4:0 (butyric acid) | 3.6 | 3.3 | 4.1 |
| c6:0 (caproic acid) | 2.2 | 1.9 | 2.4 |
| c8:0 (caprylic acid) | 1.2 | 1.1 | 1.4 |
| c10:0 (capric acid) | 2.6 | 2.2 | 2.8 |
| c10:1 (2-decenoate) | 0.3 | 0.3 | 0.3 |
| c12:0 (lauric acid) | 2.9 | 2.5 | 3.2 |
| c12:1 (11-dodecenoic acid) | 0.1 | 0.1 | 0.1 |
| c13:0 br (tridecanoic acid br) | 0.1 | 0.1 | 0.1 |
| c13:0 (tridecanoic acid) | 0.1 | 0.1 | 0.1 |
| c14:0 br (myristic acid br) | 0.2 | 0.1 | 0.2 |
| c14:0 (myristic acid) | 10.4 | 9.5 | 10.8 |
| c14:1 (myristoleic acid) | 0.9 | 0.6 | 1.0 |
| c15:0 iso br | 0.4 | 0.3 | 0.5 |
| c15:0 ante-iso br | 0.6 | 0.5 | 0.7 |
| c15:0 (pentadecanoic acid) | 1.4 | 1.1 | 1.5 |
| c16:0 br | 0.3 | 0.2 | 0.3 |
| c16:0 (palmitic acid) | 28.7 | 25.4 | 30.4 |
| c16:1 (palmitoleic acid) | 1.9 | 1.6 | 2.0 |
| c17:0 iso br | 0.7 | 0.6 | 0.7 |
| c17:0 ante-iso br | 0.5 | 0.5 | 0.5 |
| c17:0 (margaric acid) | 0.7 | 0.6 | 0.8 |
| c17:1 | 0.3 | 0.3 | 0.4 |
| c18:0 (stearic acid) | 11.5 | 10.8 | 13.6 |
| c18:1 (oleic acid) | 23.4 | 21.8 | 26.4 |
| c18:2 (linoleic acid) | 1.4 | 1.3 | 1.7 |
| c18:2 conj | 1.3 | 1.0 | 1.8 |
| c18:3 | 0.8 | 0.7 | 0.9 |
| c20:0 (arachidic acid) | 0.2 | 0.1 | 0.2 |
| c20:1 | 0.3 | 0.2 | 0.3 |

**Table 2: Exemplary Hard Fraction (Fraction H) composition**

| **Fatty acid component** | **Mean (% w/w)** | **Min (% w/w)** | **Max (% w/w)** |
|---|---|---|---|
| c4:0 (butyric acid) | 2.0 | 1.8 | 2.1 |
| c6:0 (caproic acid) | 1.3 | 1.2 | 1.4 |
| c8:0 (caprylic acid) | 0.8 | 0.8 | 0.9 |
| c10:0 (capric acid) | 2.2 | 1.9 | 2.4 |
| c10:1 (2-decenoate) | 0.2 | 0.1 | 0.2 |
| c12:0 (lauric acid) | 3.0 | 2.6 | 3.4 |
| c12:1 (11-dodecenoic acid) | 0.0 | 0.0 | 0.1 |
| c13:0 br (tridecanoic acid br) | 0.1 | 0.1 | 0.1 |
| c13:0 (tridecanoic acid) | 0.1 | 0.1 | 0.1 |
| c14:0 br (myristic acid br) | 0.1 | 0.1 | 0.2 |
| c14:0 (myristic acid) | 11.8 | 10.7 | 12.6 |
| c14:1 (myristoleic acid) | 0.6 | 0.3 | 0.7 |
| c15:0 iso br | 0.4 | 0.3 | 0.5 |
| c15:0 ante-iso br | 0.5 | 0.4 | 0.6 |
| c15:0 (pentadecanoic acid) | 1.6 | 1.2 | 1.7 |
| c16:0 br | 0.3 | 0.3 | 0.3 |
| c16:0 (palmitic acid) | 34.8 | 31.5 | 36.6 |
| c16:1 (palmitoleic acid) | 1.3 | 1.1 | 1.6 |
| c17:0 iso br | 0.8 | 0.7 | 0.8 |
| c17:0 ante-iso br | 0.5 | 0.5 | 0.6 |
| c17:0 (margaric acid) | 0.9 | 0.8 | 0.9 |
| c17:1 | 0.2 | 0.2 | 0.3 |
| c18:0 (stearic acid) | 15.2 | 13.9 | 19.7 |
| c18:1 (oleic acid) | 17.0 | 15.5 | 19.8 |
| c18:2 (linoleic acid) | 1.3 | 1.1 | 1.5 |
| c18:2 conj | 0.8 | 0.6 | 1.1 |
| c18:3 | 0.5 | 0.4 | 0.6 |
| c20:0 (arachidic acid) | 0.2 | 0.2 | 0.3 |
| c20:1 | 0.2 | 0.1 | 0.2 |

**Table 3: Exemplary Soft Hard Fraction (Fraction SH) composition**

| **Fatty acid component** | **Mean (% w/w)** | **Min (% w/w)** | **Max (% w/w)** |
|---|---|---|---|
| c4:0 (butyric acid) | 4.0 | 3.7 | 4.3 |
| c6:0 (caproic acid) | 2.4 | 2.1 | 2.6 |
| c8:0 (caprylic acid) | 1.2 | 1.1 | 1.4 |
| c10:0 (capric acid) | 2.4 | 2.2 | 2.7 |
| c10:1 (2-decenoate) | 0.3 | 0.2 | 0.3 |
| c12:0 (lauric acid) | 2.5 | 2.3 | 2.7 |
| c12:1 (11-dodecenoic acid) | 0.1 | 0.0 | 0.1 |
| c13:0 br (tridecanoic acid br) | 0.1 | 0.1 | 0.1 |
| c13:0 (tridecanoic acid) | 0.1 | 0.1 | 0.1 |
| c14:0 br (myristic acid br) | 0.1 | 0.1 | 0.2 |
| c14:0 (myristic acid) | 9.8 | 9.0 | 10.3 |
| c14:1 (myristoleic acid) | 0.8 | 0.5 | 0.9 |
| c15:0 iso br | 0.4 | 0.3 | 0.4 |
| c15:0 ante-iso br | 0.5 | 0.4 | 0.6 |
| c15:0 (pentadecanoic acid) | 1.4 | 1.1 | 1.5 |
| c16:0 br | 0.2 | 0.2 | 0.3 |
| c16:0 (palmitic acid) | 32.8 | 29.8 | 34.0 |
| c16:1 (palmitoleic acid) | 1.5 | 1.3 | 1.8 |
| c17:0 iso br | 0.6 | 0.6 | 0.7 |
| c17:0 ante-iso br | 0.4 | 0.4 | 0.5 |
| c17:0 (margaric acid) | 0.8 | 0.8 | 0.9 |
| c17:1 | 0.3 | 0.2 | 0.3 |
| c18:0 (stearic acid) | 13.2 | 12.5 | 16.1 |
| c18:1 (oleic acid) | 19.5 | 17.4 | 22.2 |
| c18:2 (linoleic acid) | 1.3 | 1.2 | 1.5 |
| c18:2 conj | 1.2 | 1.0 | 1.6 |
| c18:3 | 0.7 | 0.6 | 0.7 |
| c20:0 (arachidic acid) | 0.2 | 0.2 | 0.3 |
| c20:1 | 0.2 | 0.2 | 0.3 |

**Table 4: Exemplary Soft Soft Hard Fraction (Fraction SSH) composition**

| **Fatty acid component** | **Mean (% w/w)** | **Min (% w/w)** | **Max (% w/w)** |
|---|---|---|---|
| c4:0 (butyric acid) | 4.0 | 3.9 | 4.3 |
| c6:0 (caproic acid) | 2.4 | 2.2 | 2.6 |
| c8:0 (caprylic acid) | 1.4 | 1.2 | 1.6 |
| c10:0 (capric acid) | 2.8 | 2.4 | 3.4 |
| c10:1 (2-decenoate) | 0.3 | 0.3 | 0.3 |
| c12:0 (lauric acid) | 3.2 | 2.7 | 3.8 |
| c12:1 (11-dodecenoic acid) | 0.1 | 0.1 | 0.1 |
| c13:0 br (tridecanoic acid br) | 0.1 | 0.1 | 0.1 |
| c13:0 (tridecanoic acid) | 0.1 | 0.1 | 0.1 |
| c14:0 br (myristic acid br) | 0.2 | 0.1 | 0.2 |
| c14:0 (myristic acid) | 11.5 | 10.6 | 12.2 |
| c14:1 (myristoleic acid) | 0.9 | 0.7 | 1.0 |
| c15:0 iso br | 0.4 | 0.4 | 0.5 |
| c15:0 ante-iso br | 0.6 | 0.6 | 0.7 |
| c15:0 (pentadecanoic acid) | 1.4 | 1.2 | 1.5 |
| c16:0 br | 0.3 | 0.2 | 0.3 |
| c16:0 (palmitic acid) | 28.6 | 25.7 | 30.0 |
| c16:1 (palmitoleic acid) | 1.8 | 1.6 | 2.0 |
| c17:0 iso br | 0.7 | 0.6 | 0.7 |
| c17:0 ante-iso br | 0.5 | 0.5 | 0.5 |
| c17:0 (margaric acid) | 0.7 | 0.6 | 0.8 |
| c17:1 | 0.3 | 0.3 | 0.4 |
| c18:0 (stearic acid) | 10.6 | 10.2 | 11.3 |
| c18:1 (oleic acid) | 22.2 | 20.3 | 24.8 |
| c18:2 (linoleic acid) | 1.4 | 1.3 | 1.5 |
| c18:2 conj | 1.3 | 1.1 | 1.7 |
| c18:3 | 0.8 | 0.8 | 1.0 |
| c20:0 (arachidic acid) | 0.2 | 0.1 | 0.2 |
| c20:1 | 0.2 | 0.0 | 0.3 |

**Table 5: Exemplary Soft Soft Soft Fraction (Fraction SSS) composition**

| **Fatty acid component** | **Mean (% w/w)** | **Min (% w/w)** | **Max (% w/w)** |
|---|---|---|---|
| c4:0 (butyric acid) | 4.4 | 4.0 | 4.7 |
| c6:0 (caproic acid) | 2.7 | 2.4 | 2.8 |
| c8:0 (caprylic acid) | 1.6 | 1.4 | 1.8 |
| c10:0 (capric acid) | 3.4 | 2.8 | 3.7 |
| c10:1 (2-decenoate) | 0.4 | 0.3 | 0.4 |
| c12:0 (lauric acid) | 3.7 | 3.2 | 4.1 |
| c12:1 (11-dodecenoic acid) | 0.1 | 0.1 | 0.1 |
| c13:0 br (tridecanoic acid br) | 0.2 | 0.1 | 0.2 |
| c13:0 (tridecanoic acid) | 0.1 | 0.1 | 0.1 |
| c14:0 br (myristic acid br) | 0.2 | 0.2 | 0.2 |
| c14:0 (myristic acid) | 10.2 | 9.5 | 11.0 |
| c14:1 (myristoleic acid) | 1.2 | 0.8 | 1.3 |
| c15:0 iso br | 0.5 | 0.4 | 0.5 |
| c15:0 ante-iso br | 0.8 | 0.7 | 0.9 |
| c15:0 (pentadecanoic acid) | 1.1 | 0.9 | 1.2 |
| c16:0 br | 0.3 | 0.2 | 0.3 |
| c16:0 (palmitic acid) | 20.0 | 18.4 | 21.1 |
| c16:1 (palmitoleic acid) | 2.6 | 2.2 | 3.0 |
| c17:0 iso br | 0.6 | 0.5 | 0.6 |
| c17:0 ante-iso br | 0.5 | 0.5 | 0.5 |
| c17:0 (margaric acid) | 0.4 | 0.4 | 0.5 |
| c17:1 | 0.5 | 0.5 | 0.6 |
| c18:0 (stearic acid) | 6.7 | 5.8 | 7.8 |
| c18:1 (oleic acid) | 30.8 | 28.2 | 33.4 |
| c18:2 (linoleic acid) | 1.9 | 1.7 | 2.1 |
| c18:2 conj | 1.7 | 1.3 | 2.3 |
| c18:3 | 1.3 | 1.1 | 1.4 |
| c20:0 (arachidic acid) | 0.1 | 0.1 | 0.1 |
| c20:1 | 0.3 | 0.1 | 0.4 |

The term "beta-serum" means an aqueous dairy ingredient separated from dairy streams containing greater than 60% fat which have been through phase inversion from an oil-in-water to a water-in-oil emulsion, as described below. Cream is the preferred starting material for the production of beta-serum. For example beta-serum is produced during the production of butter-oil (also known as anhydrous milk fat or AMF) from cream as shown in Figure 2 of WO 2006/041316. Preferably the beta serum is dried; preferably dried beta-serum is a powder.

The term "high CLA milk fat" is used interchangeably with the term "CLA-enriched milk fat" and means milk fat that comprises a higher level of c-9, t-11 CLA or a salt, ester or precursor thereof than normal milk fat, and, optionally, a higher level of one or more other CLA isomers. CLA-enriched milk fat may prepared by known techniques including but not limited to supplementary free fatty acid feeding of pasture fed cows by, for example, feeding cows with fish oil and sunflower oil according to known methods. CLA-enriched milk fat is typically prepared from whole milk but may also be prepared from colostrum. A typical composition of CLA-enriched milk fat is described in published international PCT application WO 2005/107736 that is hereby incorporated by reference. CLA-enriched milk fat may also be prepared by supplementing milk fat with CLA, as described below. In one embodiment the CLA-enriched milk fat comprises at least about 2, 4, 6, 8, 10, 15, 20, 25, 30, 35, 40, 45 or 50% by weight CLA, preferably c-9, t-11 CLA or a salt, ester or precursor thereof and useful ranges may be selected between any of these forgoing values (e.g. from about 4% to about 7%). Preferably the CLA-enriched milk fat comprises at least about 2% c-9, t-11 CLA by weight, preferably about 2 to 10% c-9, t-11 CLA by weight, more preferably about 4 to 7% c-9, t-11 CLA by weight and most preferably about 5% c-9, t-11 CLA by weight. In one embodiment the CLA-enriched milk fat comprises CLA isomers which comprise at least about 50, 55, 60, 65, 70, 75, 80, 85, 90, 95 or 99% by weight CLA, preferably c-9, t-11 CLA or a salt, ester or precursor thereof and useful ranges may be selected between any of these forgoing values (e.g. from about 80% to about 95%). Preferably the CLA-enriched milk fat includes CLA isomers comprising at least about 50% c-9, t-11 CLA by weight, preferably about 70 to 80% c-9, t-11 CLA by weight.

The term "comprising" as used in this specification means "consisting at least in part of". When interpreting statements in this specification which include that term, the features, prefaced by that term in each statement or claim, all need to be present but other features can also be present. Related terms such as "comprise" and "comprised" are to be interpreted in the same manner.

The term "conjugated linoleic acid" (CLA) means one or more CLA isomers selected from isomers of 9,11-octadecadienoic acid and 10,12-octadecadienoic acid, in free or esterified form, or salts thereof, or mixtures thereof, including the cis-9,cis-11, cis-9,trans-11, trans-9,cis-11, trans-9,trans-11, cis-10,cis-12, cis-10,t12, trans-10,cis-12, and trans-10,t12 isomers, preferably the cis-9,cis-11, cis-9,trans-11, trans-10,cis-12, and cis-10,cis-12 isomers, as described in published United States patent US 5,585,400 incorporated herein by reference. Natural sources of CLA such as cis-9, trans-11 CLA are described by Chin et al (1992) and include animal, bacterial and plant sources. Linoleic acid may be converted to CLA by bacterial fermentation with *Clostridium sporogenes, Clostridium bifermentans, Clostridium sordellii* and *Bacteroides sp,* for example (Verhulst, et al., 1985). Other useful organisms for bacterial fermentation include *Butyrivibrio fibrisolvens, Eubacterium lentum, Propionibacterium freudenreichi, Lactobacillus acidophilus, Lactobacillus reuteri, Megasphaera elsdenii,* and *Bifidobacterium breve.* Sunflower and safflower oils, containing approximately 65% and 76% linoleic acid respectively, may be used for CLA production.

An "effective amount" is the amount required to confer therapeutic effect. The interrelationship of dosages for animals and humans (based on milligrams per meter squared of body surface) is described by Freireich, et al. (1966). Body surface area can be approximately determined from height and weight of the subject. See, e.g., Scientific Tables, Geigy Pharmaceuticals, Ardley, New York, 1970, 537. Effective doses also vary, as recognized by those skilled in the art, dependent on route of administration, carrier usage, and the like.

The terms "enrich" and "enriched" mean that the fraction or composition has a higher concentration of the named component than is present in whole milk, cream, butter, anhydrous milk fat, buttermilk, butter serum, or beta serum, or the parent fraction from which the fraction or composition is derived. For example, a ganglioside-enriched fraction is a fraction that has a higher ganglioside concentration than whole milk, cream, butter, anhydrous milk fat, buttermilk, butter serum, or beta serum. Equally, a phospholipid-enriched fraction is a fraction that has a higher phospholipid concentration than whole milk, cream, butter, anhydrous milk fat, buttermilk, butter serum, or beta serum.

The term "fraction" means a composition that has been isolated from a source material and that is compositionally different to the source material that the fraction was isolated from. For example, a non-human mammalian milk fat fraction, such as a sheep, goat, pig, mouse, water buffalo, camel, yak, horse, donkey, llama, or bovine milk fat fraction, preferably a bovine milk fat fraction, differs compositionally from the naturally occurring milk fat in whole milk. In alternative embodiments the concentration in the fraction is higher than the concentration in whole milk, or in whole colostrum, or in cream from milk, or in cream from colostrum, or in AMF from milk, or AMF from colostrum. Preferred source material useful herein includes whole milk, cream, anhydrous milk fat, buttermilk, butter serum, or beta serum, or whey cream from bovine milk. Preferred fractions are lipid fractions, as described herein.

Accordingly, the term "phospholipid-enriched milk fat fraction" means an isolated fraction of non-human mammalian milk fat where the phospholipid concentration of the fraction is higher than the phospholipid concentration of naturally occurring non-human mammalian milk fat. Preferably the concentration of at least one phospholipid or at least one phospholipid and at least one ganglioside in a fraction useful herein is at least about 0.5, 1, 5, 10, 15, 20, 25, 30, 35, 40, 45, 50, 55, 60, 65, 70, 75, 80, 85, 90, 95, or 100% higher than the concentration in naturally occurring non-human mammalian milk fat, and useful ranges may be selected between these values. In alternative embodiments the concentration in the fraction is higher than the concentration in whole milk, or in whole colostrum, or in cream from milk, or in cream from colostrum, or in AMF from milk, or AMF from colostrum.

Equally, the term "ganglioside-enriched milk fat fraction" means an isolated fraction of non-human mammalian milk fat where the ganglioside concentration of the fraction is higher than the phospholipid concentration of naturally occurring non-human mammalian milk fat. Preferably the concentration of at least one ganglioside or at least one ganglioside and at least one phospholipid in a fraction useful herein is at least about 0.5, 1, 5, 10, 15, 20, 25, 30, 35, 40, 45, 50, 55, 60, 65, 70, 75, 80, 85, 90, 95, or 100% higher than the concentration in naturally occurring non-human mammalian milk fat, and useful ranges may be selected between these values. In alternative embodiments the concentration in the fraction is higher than the concentration in whole milk, or in whole colostrum, or in cream from milk, or in cream from colostrum, or in AMF from milk, or AMF from colostrum.

The term "functional lactoferrin fragment" is intended to mean a naturally occurring or non-naturally occurring portion of a lactoferrin polypeptide that has activity when assayed according the examples below, and includes metal ion functional fragments. Useful lactoferrin fragments include truncated lactoferrin polypeptides, metal ion-binding hydrolysates of lactoferrin, fragments that comprise the N-lobe metal ion binding pocket, fragments that comprise the C-lobe metal ion binding pocket, and metal ion-binding fragments generated (by artificial or natural processes) and identified by known techniques as discussed below. Published international patent applications WO 2006/054908 and WO 2007/043900 report preparation and use of lactoferrin fragments and are each incorporated herein by reference.

The term "functional lactoferrin variant" is intended to mean a variant of a lactoferrin polypeptide that has activity when assayed according the examples below, and includes metal ion functional variants.

The term "lactoferrin polypeptide" refers to a non-glycosylated or glycosylated wild-type lactoferrin amino acid sequence or a homologous lactoferrin sequence from a species such as those described below. A lactoferrin polypeptide has two metal-ion binding pockets and so can bind metal ions in a stoichiometric ratio of 2 metal ions per lactoferrin molecule. One metal ion-binding pocket is present in the N-terminal lobe (N-lobe) of lactoferrin and the other pocket is present in the C-terminal lobe (C-lobe). Verified sequences of bovine and human lactotransferrins (lactoferrin precursors), lactoferrins and peptides therein can be found in Swiss-Prot (http://au.expasy.org/cgi-bin/sprot-search-ful). Indicative lactoferrin polypeptides include the bovine lactotransferrin precursor accession number P24627, bovine lactoferrin, the human lactotransferrin precursor accession number P02788 and human lactoferrin. Published international patent applications WO 2006/054908 and WO 2007/043900 report preparation (including isolation from milk) and use of lactoferrin polypeptides, fragments, hydrolysates, and amino acid sequences thereof, and each application is incorporated herein by reference. Lactoferrin polypeptides may bind "natural" levels of metal ions, typically iron ions. For example, bovine lactoferrin is naturally about 10% to 20% (preferably 15%) iron saturated. Apo-lactoferrin and lactoferrin of at least 1 % metal ion saturation is useful herein.

The terms "metal ion lactoferrin", "metal ion-saturated lactoferrin", "metal ion lactoferrin fragment" and "metal ion-saturated lactoferrin fragment" are intended to refer to a population of lactoferrin polypeptides or fragments that provide a population of metal ion-binding pockets where at least about 25% of the metal ion-binding pockets present in the population have a metal ion bound. It should be understood that the population may contain polypeptides of different species; for example, some molecules binding no ion and others each binding one or two ions. In cases where different metal ions are used, some molecules may bind a metal ion selected from, for example, the group comprising aluminium, bismuth, copper, chromium, cobalt, gold, iron, manganese, osmium, platinum, ruthenium, zinc ions, or other ions that will coordinate specifically in a lactoferrin metal ion binding pocket, and others may bind a different ion. In some cases, the population may comprise polypeptides involved in non-specific ion binding, where one or more ions, preferably metal ions, are non-specifically bound, i.e., not bound in the metal-ion binding pocket, to the polypeptide. Non-limiting examples of ions that may be non-specifically bound to lactoferrin polypeptides are calcium and selenium. Varying degrees of saturation may be achieved and the degree of saturation may be determined by spectrophotometric analysis by known methods - see for example published international application WO 2007/043900 incorporated herein by reference. In one embodiment at least about 1, 5, 10, 15, 20, 25, 30, 35, 40, 45, 50, 55, 60, 65, 70, 75, 80, 85, 90, 91, 92, 93, 94, 95, 96, 97, 98, 99, 99.5, 99.9 or 100% of the metal ion-binding pockets present in the population of lactoferrin molecules have a metal ion bound and useful ranges may be selected between any of the foregoing values (for example, about 5 to about 100%, about 10 to about 100%, about 15 to about 100%, about 20 to about 100%, about 25 to about 100%, about 30 to about 100%, about 35 to about 100%, about 40 to about 100%, about 45 to about 100%, about 50 to about 100%, about 55 to about 100%, about 60 to about 100%, about 65 to about 100%, about 70 to about 100%, about 75 to about 100%, about 80 to about 100%, about 85 to about 100%, about 90 to about 100%, about 95 to about 100% and about 99 to about 100%).

The term "milk fat" includes mammalian milk lipids and lipid fractions, lipid hydrolysates, and lipid fraction hydrolysates. In some embodiments, milk fat may be any mammalian milk fat including but not limited to bovine, sheep, goat, pig, mouse, water buffalo, camel, yak, horse, donkey, llama or human milk fat, with bovine milk fat being a preferred source. Preferred milk fats are dairy fats, particularly bovine milk fats. Preferred milk fat has one or more of palmitic acid, oleic acid, stearic acid, or myristic acid as the most abundant fatty acid(s) present, preferably palmitic, oleic, stearic and myristic acids are the most abundant fatty acids present. In particularly preferred embodiments, the milk fat, such as cream or AMF for example, has a) substantially the same percentage by weight of palmitic acid as does normal bovine milk fat (between about 23%(w/w) and about 32%(w/w), typically about 28%(w/w) - see Table 1.2, PF Fox and PLH McSweeney eds, Advanced Dairy Chemistry Volume 2 - Lipids, 3rd Ed, Springer NY, NY (2006) ISBN-10:0-387-26364-0); b) substantially the same percentage by weight of oleic acid as does normal bovine milk fat (between about 15%(w/w) and about 22%(w/w), typically about 17%(w/w) - see Fox and McSweeny ibid); c) substantially the same percentage by weight of stearic acid as does normal bovine milk fat (between about 10%(w/w) and about 15%(w/w), typically about 12%(w/w) - see Fox and McSweeny ibid); d) substantially the same percentage by weight of myristic acid as does normal bovine milk fat (between about 9%(w/w) and about 12%(w/w), typically about 11%(w/w) - see Fox and McSweeny ibid); e) any two of a), b), c), or d) above; f) any three of a), b), c), or d) above; g) each of a), b), c), and d) above. Anhydrous milk fat (AMF) is preferred, particularly AMF having substantially the same percentage by weight palmitic, oleic and stearic acid composition as normal bovine milk fat, more preferably substantially the same fatty acid composition as normal bovine milk fat (see Fox and McSweeny ibid). Preferred milk fat fractions also include cream, butter, anhydrous milk fat (AMF) (typically produced by phase inversion of cream or dehydration of butter), butter milk, butter serum, beta serum, hard milk fat fractions from one or more stages of fractionation (including H, SH, and SSH fractions), soft milk fat fractions from one or more stages of fractionation (including S, SS, and SSS fractions), combinations of hard milk fat fractions, combinations of soft milk fat fractions, combinations of hard milk fat fractions and soft milk fat fractions, sphingolipid fractions (including sphingomyelin fractions, ceramide fractions, cerebroside fractions or ganglioside fractions, or any combination of any two or more thereof), milk fat globule membrane lipid fractions, phospholipid fractions, and complex lipid fractions, or any combination of any two or more thereof, and hydrolysates of any one or more thereof, and fractions of the hydrolysates, combinations of any two or more hydrolysates, and combinations of one or more hydrolysed and/or one or more non-hydrolysed fractions. Preferably, the milk fat comprises at least about 20, 30, 40, 50, 60, 70, 80, 85, 90, 95, 99 or 100% lipid, and useful ranges may be selected between any of these values (for example, about 60 to about 100, about 70 to about 100, about 80 to about 100, about 85 to about 100, about 90 to about 100, about 95 to about 100, about 96 to about 100, about 97 to about 100, about 98 to about 100, and about 99 to about 100%, preferably about 40% or greater to about 100%).

The term "oral administration" includes oral, buccal, enteral and intra-gastric administration.

The term "parenteral administration" includes but is not limited to topical (including administration to any dermal, epidermal or mucosal surface), subcutaneous, intravenous, intraperitoneal, and intramuscular administration.

The term "pharmaceutically acceptable carrier" is intended to refer to a carrier including but not limited to an excipient, diluent or auxiliary, or combination thereof, that can be administered to a subject as a component of a composition described herein that does not reduce the activity of the composition and is not toxic when administered in doses sufficient to deliver an effective amount of a compound or composition useful herein. The formulations can be administered orally, nasally or parenterally (including topically, intramuscularly, intraperitoneally, subcutaneously and intravenously).

A "subject" is an animal, preferably a mammal, more preferably, a mammalian companion animal or human. Preferred companion animals include cats, dogs and horses. In one embodiment the human is an adult, a child, or an infant, or an immunocompromised adult, child, or infant.

The term "treat" and its derivatives should be interpreted in their broadest possible context. The term should not be taken to imply that a subject is treated until total recovery. Accordingly, "treat" broadly includes amelioration and/or prevention of the onset of the symptoms or severity of a particular condition.

### 2. Milk fat and milk fat fractionation

Milk fat is discussed comprehensively by Fox and McSweeney (2006), hereby incorporated by reference. In addition to lipids, milk fat includes vitamins, sterols, and minor components. See Chapter 1, Composition and Structure of Bovine Milk Lipids, Fox and McSweeney, for a description of naturally occurring bovine milk fat. Fractionation of milk fat is discussed in the Dairy Processing Handbook, 1995, and by Illingworth, 2002, and by Rombaut et al, 2006(b), all hereby incorporated by reference. Seasonal variation of milk fat is discussed by Fox and McSweeney (2006).

Examples of milk fat fractions useful according to the invention include cream (typically about 20 to about 40% fat by weight, preferably about 40% fat by weight), butter, ghee, anhydrous milk fat (AMF) (typically produced by phase inversion of cream or dehydration of butter), buttermilk, butter serum, beta serum, hard milk fat fractions, soft milk fat fractions, sphingolipid fractions, milk fat globular membrane fractions, milk fat globular membrane lipid fractions, phospholipid fractions, and complex lipid (lipids that yield 3 or more types of hydrolysis product per molecule) fractions, and combinations thereof, and hydrolysates thereof.

Buttermilk, butter serum, and beta serum are discussed by Bylund, 1995, Rombaut et al, 2005, Rombaut et al, 2006(a), Rombaut et al, 2006(b), and published international application WO 2006/041316, for example, all incorporated herein by reference. Buttermilk is a term used to describe the aqueous liquid phase obtained from traditional butter production using a butter making process which may be a batch (churn) process or a continuous (Fritz) process. Buttermilk is also a term used to describe the aqueous by-product produced by the cream concentration step of the traditional method of producing AMF from cream. This traditional method involves concentration then phase inversion of cream to produce oil that is further concentrated and polished to produce AMF. Finally, buttermilk is also a term used to describe a combination of the secondary skim and beta serum by-products of a two-serum process for AMF production - see for example, Bylund (Ed., 1995) and published international application WO 2006/041316 (see Figure 2) that describe this process in detail. In that two-serum process, the by-product from the cream concentration step is further separated to produce secondary skim and the by-product from the oil concentration step is further separated to produce beta-serum. In the first two instances, the buttermilk is produced before any phase inversion has occurred. In the third instance, the buttermilk is a combination of secondary skim produced before phase inversion and beta serum produced after phase inversion. Concentration and polishing in these processes is typically achieved by centrifugation. Phase inversion is typically achieved by homogenisation. It should be understood that the source of these dairy lipid fractions may be milk or colostrum or a combination thereof.

Useful starting materials for fractionation include cream, AMF, butter milk, butter serum, or beta serum, from milk or colostrum or a combination thereof.

Multistage fractionation of milk fat may be carried out by differential crystallisation. Milk fat fractions are heated to a set temperature and the crystallised or solid ("stearin" - hard fraction) and liquid ("olein" - soft fraction) fractions are separated. Multi-step fractionation refers to re-fractionation in a subsequent step of a product of a previous fractionation step. Successive soft fractions may be produced by fractionating parent soft fractions into soft and hard sub-fractions.

Other fractionation methods include phase inversion, interesterification, glycerolysis, solvent fractionation (such as with ethanol, water, or acetone, used alone or sequentially), supercritical fractionation (see Astaire, et al, 2003, for example), near critical fractionation (see WO 2004/066744, for example), distillation, centrifugal fractionation, suspension crystallisation, dry crystallisation, fractionation with a modifier (e.g. soaps or emulsifiers), ultra-filtration, micro-filtration, and any process for fractionation of lipid known in the art, and combinations of these methods, all as known in the art.

In one embodiment, the fractionation method is selected from solvent fractionation of cream, AMF, butter milk, butter serum, or beta serum, using ethanol, water, or acetone, alone or sequentially.

Lipids present in the compositions of the invention may be fully or partially modified, whether naturally, chemically, enzymatically, or by any other methods known in the art, including, for example, glycosylated, sialylated, esterified, phosphorylated or hydrolysed. Lipid hydrolysates may be prepared using known techniques, including but not limited to acid hydrolysis, base hydrolysis, enzymatic hydrolysis using a lipase, for example as described in Fox and McSweeney ((2006), Chapter 15 by HC Deeth and CH Fitz-Gerald), and microbial fermentation. One method of base hydrolysis includes adding 1% KOH (in ethanol) and heating for 10 minutes. Hydrolysed material may be neutralised with acetic acid or hydrochloric acid.

Milk fat globule membrane material may be isolated according to the acidification method of Kanno & Dong-Hyun, 1990, and further fractionated into lipid and protein fractions by the addition of methanol, as described by Kanno et al, 1975. A phospholipid fraction may be isolated by extracting the lipid mixture with acetone according to the procedure of Purthi et al, 1970. Lipid residue may be further enriched in milk fat globule membrane lipids by the selective extraction of non-polar lipids with pentane.

Fractionation methods useful to produce milk fat fractions useful herein are also described in published international patent applications WO 2006/041316, WO 2007/123424, and WO 2007/123425 that are each hereby incorporated herein by reference in their entirety.

Particularly preferred milk fat fractions useful herein include those described in the examples below and those summarised in the following tables. These fractions may be emulsions or dried, and may be powders, optionally with components including flow aids such as lactose added to improve flowability.

**Table 6 - Milk fat fractions**

| | **Fraction** | | | | | |
|---|---|---|---|---|---|---|
| | **1** | **2** | **3** | **4** | **5** | **6** |
| **Component (%w/w)** | **H** | **SH** | **SSH** | **S** | **SS** | **SSS** |
| Protein | <0.01 | <0.01 | <0.01 | <0.01 | <0.01 | <0.01 |
| Fat (neutral lipid) | 99.9 | 99.9 | 99.9 | 99.9 | 99.9 | 99.9 |
| Phospholipid | <0.01 | <0.01 | <0.01 | <0.01 | <0.01 | <0.01 |
| Lactose | <0.01 | <0.01 | <0.01 | <0.01 | <0.01 | <0.01 |
| Ash | <0.01 | <0.01 | <0.01 | <0.01 | <0.01 | <0.01 |
| Moisture | <0.1 | <0.1 | <0.1 | <0.1 | <0.1 | <0.1 |

| | | | | | | |
|---|---|---|---|---|---|---|
| <0.01 = trace amounts | | | | | | |

**Table 7 - Phospholipid and ganglioside fractions**

| | **Fraction** | | | | | | | | | | |
|---|---|---|---|---|---|---|---|---|---|---|---|
| | **7** | **8** | **9** | **10** | **11** | **12** | **13** | **14** | **15** | **16** | **17** |
| **Component (%w/w)** | **beta serum** | | | | | | **PC500™** | **PC700™** | | **G500™** | **G600™** |
| Protein | 30.2 | 49.7 | 60.2 | <0.01 | <0.01 | 12.4 | 0.0 | 0.0 | ND | <2% | 10.2 |
| MFGM | 7.5 | 11.9 | 14.4 | 0.2 | ND | ND | 0.0 | 0.0 | ND | ND | ND |
| Fat | 20.6 | 35.6 | 23.1 | 94.2 | 86.8 | 90.2 | 87.0 | 84.4 | 84.6 | 35.5 | 27.9 |
| Phospholipid | 9.7 | 14.9 | 16.0 | 31.0 | 65.7 | 66.8 | 24.7 | 60.2 | 27.6 | 17.6 | 15.1 |
| PC | 2.5 | 3.8 | 4.9 | 8.1 | 16.8 | 15.0 | 8.0 | 19.2 | 3.2 | 3.1 | 2.0 |
| PI | 0.8 | 1.1 | 1.5 | 2.8 | 5.8 | 6.0 | 0.7 | 2.0 | 6.0 | 2.8 | 2.9 |
| PS | 1.1 | 1.6 | 2.1 | 4.3 | 8.7 | 7.6 | 1.0 | 2.4 | 7.3 | 3.5 | 4.0 |
| PE | 2.8 | 4.3 | 5.4 | 11.3 | 23.6 | 21.8 | 7.7 | 17.0 | 6.4 | 4.9 | 4.4 |
| SM | 2.4 | 3.6 | 4.5 | 7.5 | 16.5 | 13.6 | 6.9 | 16.7 | 3.5 | 2.8 | 1.6 |
| Gangliosides | 0.4 | 0.7 | 1.0 | 1.2 | 2.0 | 2.0 | 0.0 | 0.0 | 4.5 | 1.3 | 2.0 |
| GD3 | 0.4 | 0.6 | 0.9 | 1.1 | 1.8 | 1.8 | 0.0 | 0.0 | 4.0 | 0.6 | 1.8 |
| Lactose | ND | 7.8 | 11.7 | 2.6 | 6.4 | 4.0 | 4.1 | 6.2 | 8.3 | 54.9 | 58.0 |
| Ash | ND | 5.2 | 5.9 | 3.1 | 12.1 | 9.1 | 13.3 | 7.4 | 7.0 | 5.0 | 8.3 |
| Moisture | 1.9 | 2.7 | 2.9 | 2.6 | 4.6 | 2.3 | 2.2 | 2.0 | 3.7 | 3.2 | 2.8 |

| | | | | | | | | | | | |
|---|---|---|---|---|---|---|---|---|---|---|---|
| ND = not determined; <0.01 = trace amounts | | | | | | | | | | | |

### 3. Compositions useful according to invention

A composition useful herein may be formulated as a food, drink, food additive, drink additive, dietary supplement, nutritional product, medical food, enteral or parenteral feeding product, meal replacement, cosmeceutical, or pharmaceutical. Appropriate formulations may be prepared by an art skilled worker with regard to that skill and the teaching of this specification.

In one embodiment, compositions useful herein include any edible consumer product which is able to carry lipid. Examples of suitable edible consumer products include powders, liquids, confectionary products including chocolate, gels, ice creams, reconstituted fruit products, snack bars, food bars, muesli bars, spreads, sauces, dips, dairy products including yoghurts and cheeses, drinks including dairy and non-dairy based drinks (such as milk drinks and yogurt drinks), milk powders, sports supplements including dairy and non-dairy based sports supplements, food additives such as protein sprinkles, dietary supplement products including daily supplement tablets, weaning foods and yoghurts, and formulas such as infant formula, follow-on formula, or growing-up formula, in powder or liquid form. Within this embodiment, a preferred composition useful herein may be an infant formula, follow-on formula or growing-up formula, in powder or liquid form. Suitable nutraceutical compositions useful herein may be provided in similar forms.

Examples of formulas such as infant formula, follow-on formula, or growing-up formula, in powder or liquid form, include the following. One example of an infant formula, follow-on formula or growing-up formula useful herein comprises (w/w)
(a) 30 - 60 % lactose
(b) 15 - 35% vegetable oils
(c) 0 - 40% skim milk powder
(d) 0 - 40% whey protein, such as a WPC or WPI, preferably an 80% WPC (WPC80)
(e) 1 - 50% of an agent useful herein.

Another example of an infant formula, follow-on formula or growing-up formula useful herein comprises (w/w)
(a) 40 - 60 % lactose
(b) 20 - 30% vegetable oils
(c) 10 - 15% skim milk powder
(d) 6 - 8% whey protein, preferably WPC80
(e) 1 - 10% of an agent useful herein.

Another example of an infant formula, follow-on formula or growing-up formula useful herein comprises (w/w)
(a) 40 - 60 % lactose
(b) 20 - 30% vegetable oils
(c) 10 - 15% skim milk powder
(d) 6 - 8% whey protein, preferably WPC80
(e) 1 - 5% of an agent useful herein.

Another example of an infant formula, follow-on formula or growing-up formula useful herein comprises (w/w)
(a) 40 - 60 % lactose
(b) 20 - 30% vegetable oils
(c) 10 - 15% skim milk powder
(d) 6 - 8% whey protein, preferably WPC80
(e) 2 - 5% of an agent useful herein.

Another example of an infant formula, follow-on formula or growing-up formula useful herein comprises (w/w)
(a) 30 - 60 % lactose
(b) 15 - 35% vegetable oils
(c) 0 - 40% skim milk powder
(d) 0 - 40% whey protein, preferably WPC80
(e) 1 - 50% of an agent useful herein such as beta serum powder or a fraction thereof, such as a fraction obtained from beta serum enriched in polar lipids or depleted in neutral lipids or both.

Another example of an infant formula, follow-on formula or growing-up formula useful herein comprises (w/w)
(a) 40 - 60 % lactose
(b) 20 - 30% vegetable oils
(c) 10 - 15% skim milk powder
(d) 6 - 8% whey protein, preferably WPC80
(e) 1 - 5% of an agent useful herein such as beta serum powder or a fraction thereof, such as a fraction obtained from beta serum enriched in polar lipids or depleted in neutral lipids or both.

Any of these infant formulas may also comprise 0.1 to 4% w/w, preferably 2 to 4% w/w/ of one or more of a vitamin premix, a mineral premix, lecithin, one or more antioxidants, one or more stabilisers, or one or more nucleotides, or a combination of any two or more thereof. In some embodiments, these infant formulas may be formulated to provide between 2700 and 3000 kJ/L.

In alternative embodiments, the compositions useful herein may be formulated to allow for administration to a subject by any chosen route, including but not limited to oral or parenteral (including topical, subcutaneous, intramuscular and intravenous) administration.

Thus, a pharmaceutical composition useful according to the invention may be formulated with an appropriate pharmaceutically acceptable carrier (including excipients, diluents, auxiliaries, and combinations thereof) selected with regard to the intended route of administration and standard pharmaceutical practice. For example, a composition useful according to the invention can be administered orally as a powder, liquid, tablet or capsule, or topically as an ointment, cream or lotion. Suitable formulations may contain additional agents as required, including emulsifying, antioxidant, flavouring or colouring agents, and may be adapted for immediate-, delayed-, modified-, sustained-, pulsed- or controlled-release.

Capsules can contain any standard pharmaceutically acceptable materials such as gelatin or cellulose. Tablets can be formulated in accordance with conventional procedures by compressing mixtures of the active ingredients with a solid carrier and a lubricant. Examples of solid carriers include starch and sugar bentonite. Active ingredients can also be administered in a form of a hard shell tablet or a capsule containing a binder, e.g., lactose or mannitol, a conventional filler, and a tabletting agent. Pharmaceutical compositions can also be administered via the parenteral route. Examples of parenteral dosage forms include aqueous solutions, isotonic saline or 5% glucose of the active agent, or other well-known pharmaceutically acceptable excipients. Cyclodextrins, or other solubilising agents well-known to those familiar with the art, can be utilized as pharmaceutical excipients for delivery of the therapeutic agent.

The efficacy of a composition useful according to the invention can be evaluated both *in vitro* and *in vivo.* See, e.g., the examples below. Briefly, the composition can be tested for its ability to inhibit rotavirus infection *in vitro.* For *in vivo* studies, the composition can be fed to or injected into an animal (e.g., a mouse) and its effects on viral infection are then assessed. Based on the results, an appropriate dosage range and administration route can be determined.

The compositions useful herein may be used alone or in combination with one or more other therapeutic agents. The therapeutic agent may be a food, drink, food additive, drink additive, food component, drink component, dietary supplement, nutritional product, medical food, nutraceutical, medicament or pharmaceutical. The therapeutic agent is preferably effective to attenuate one or more of the symptoms of a rotavirus infection.

When used in combination with another therapeutic agent, the administration of a composition useful herein and the other therapeutic agent may be simultaneous or sequential. Simultaneous administration includes the administration of a single dosage form that comprises all components or the administration of separate dosage forms at substantially the same time. Sequential administration includes administration according to different schedules, preferably so that there is an overlap in the periods during which the composition useful herein and other therapeutic agent are provided.

Suitable agents with which the compositions useful herein can be co-administered include anti-viral agents, theaflavins, protease inhibitors, egg yolk immunoglobulin, synthetic sulphated sialyl lipid NMSO₃, human milk lactadherin (a glycoprotein of the milk fat globule membrane), MUC1 mucin, bovine macromolecular whey protein fraction, and combinations thereof, and other suitable agents known in the art.

In one embodiment, a composition useful herein includes or is administered simultaneously or sequentially with milk components such as whey protein, whey protein fractions (including acidic or basic whey protein fractions or a combination thereof), glycomacropeptide, lactoferrin, iron-lactoferrin, a functional lactoferrin variant, a functional lactoferrin fragment, a vitamin D or calcium, or combinations thereof. Useful milk component-containing compositions include compositions such as a food, drink, food additive, drink additive, dietary supplement, nutritional product, medical food or nutraceutical. Milk fractions enriched for these components may also be employed. Useful lactoferrins, fragments and compositions are described in international patent applications WO 03/082921 and WO 2007/043900, both incorporated herein by reference in their entirety.

It should be understood that the additional therapeutic agents listed above (both food based and pharmaceutical agents) may also be employed in a method according to the invention where they are administered separately, simultaneously or sequentially with a composition useful herein.

As will be appreciated, the dose of the composition administered, the period of administration, and the general administration regime may differ between subjects depending on such variables as the severity of symptoms of a subject, the type of disorder to be treated, die mode of administration chosen, and the age, sex and/or general health of a subject. However, by way of general example, the inventors contemplate administration of from about 1 mg to about 1000 mg per kg body weight of a composition useful herein is administered per day, preferably about 50 to about 500 mg per kg per day, alternatively about 150 to about 410 mg/kg/day or about 110 to about 310 mg/kg/day. In one embodiment, the inventors contemplate administration of from about 0.05 mg to about 250 mg per kg body weight of a pharmaceutical composition useful herein.

It should be appreciated that administration may include a single daily dose or administration of a number of discrete divided doses as may be appropriate.

Various aspects of the invention will now be illustrated in non-limiting ways by reference to the following examples.

### EXAMPLES

### Sample analysis

Proteins levels were determined by Total nitrogen multiplied by 6.38. Phospholipid levels were determined by ³¹P NMR. Ganglioside levels were determined as follows. In triplicate approximately 0.1g of powder was weighed into a 16 ml kimax tube and the weight recorded. 6ml of methanol was added and mixed by vortexing for 1 min. The solution was incubated at 50°C for 10 min then 6 ml water was added and mixed by vortexing. The solution was allowed to stand for 2 hrs at 4°C to setde and a sample was taken and passed through a 0.45µm filter. The sample was analysed by HPLC. A Cosmosil™ 5NH2-MS waters column (Nacalai Tesque Inc, USA) was used with a NH2 security guard (Phenomenex™ AJO-4302 in a Phenomenex™ KJO-4282 holder). The guard cartridge was changed every day of analysis. Injections of sample were injected onto the column and eluted at a flow rate of 2ml/min using solvent A (90% acetonitrile, 5% water and 5% 5mM phosphate buffer pH5.6) and solvent B (50% acetonitrile, 45% water and 5% 200mM phosphate buffer pH5.6) The following Gradient was used: 100% A for 3.5 min, then 100% A to 55% A over 26.5 min, then 55% A to 100% A over 1 min and then 100% A for 5 min (Wagener et al. (1996), Journal of Lipid Research 37, 1823-1829). An external standard curve of 0-2 ug GD3 was generated using buttermilk GD3 (Matreya #1504). Elution was monitored at 203nm.

### Lipid preparations and other dairy materials

Milk fat fractions (including beta serum), lactoferrin, Sialyl Oligolac™ milk extract, and milk fat globule membrane (MFGM) materials were provided by Fonterra Co-operative Group Limited (New Zealand). Sialyl Oligolac™ milk extract is a spray dried powder derived from colostrum containing high levels of natural milk oligosaccharides, minerals, and free and carbohydrate-bound sialic acid (protein (as is) 8.7%, lactose 61.7%, moisture 3.9%, fat 0 08%, ash 18.9%, free sialic acid 25 mg/g, bound sialic acid 9.3 mg/g) Sialyl Oligolac™ contains 3' sialyl lactose and disialyl lactose that are the free forms of the glycan from the gangliosides GM3 and GD3 respectively The MFGM and MFGM protein fractions in Table 9 were produced according to known methods from New Zealand milk fat.

### AMF and AMF fractions

AMF, AMF hard fractions (SH and SSH), and the AMF soft fraction (SSS) provided by Fonterra Co-operative Group Limited are described above.

High CLA milk fat contains 5 4% CLA was prepared by feeding cows with fish oil and sunflower oil according to known methods and then preparing anhydrous milk fat from the milk produced A typical composition of CLA-enriched milk fat is described in published international PCT application WO 2005/107736 that is hereby incorporated by reference.

### Phospholipid fractions

Fractions 7 to 11 described in Table 7 above and used in the examples below were produced according to the methods described in published international patent application WO 2006/041316 (see examples 3 to 6). Fraction 12 was produced by supercritical carbon dioxide extraction of phospholipid fraction 10 in Table 7 Fraction 15 was produced by ethanol extraction of beta serum powder

The Phospholipid Concentrate PC500™ phospholipid fraction (available from Fonterra Co-operative Group Limited, New Zealand) is produced by ethanol extraction of beta serum powder Beta serum is the liquid phase produced during AMF manufacture. The PC500™ phospholipid fraction is a spray dried milk phospholipid concentrate with a typical composition of 77-95% total lipids, from about 50% neutral lipids and from about 30% polar lipids; and a typical lipid composition of 1.5-5% phosphatidyl serine, 12-18% phosphatidyl choline, 6-9% phosphatidyl ethanolamine, and 6.7-9% sphingomyelin; and a typical fatty acid composition of butyric acid (4:0) 1 8%, capric acid (10:0) 0.3%, lauric acid (12:0) 0 5%, myristic acid (14:0) 7.4%, myristoleic acid (14:1) 14.1%, pentadecanoic acid (15:0) 10%, palmitic acid (16:0) 26.0%, palmitoleic acid (16:1) 1 7%, margaric acid (17:0) 0.6%, heptadecenoic acid (17:1) 0.3%, stearic acid (18:0) 11.9%, oleic acid (18:1) 39.0%, linoleic acid (18:2) 5.0%, linolenic (18:3) 2.0%, arachidic acid (20:0) 0.3%, and cholesterol 0.8%.

The Phospholipid Concentrate PC600™ phospholipid fraction (available from Fonterra Co-operative Group Limited, New Zealand) is produced by acetone extraction from the PC500™ phospholipid fraction. The PC600™ phospholipid fraction is a freeze dried milk phospholipid concentrate with a typical composition of 75% polar lipids, 8.0% neutral lipids, <12% ash, and <4% moisture; a typical lipid composition of phosphatidyl serine 3-4%, phosphatidyl choline >36%, phosphatidyl ethanolamine >9%, and sphingomyelin >18%; and a typical fatty acid composition of myristic acid (14:0) 6.6%, palmitic acid (16:0) 27.1%, palmitoleic acid (16:1) 1.3%, margaric acid (17:0) 2.3%, stearic acid (18:0) 14%, oleic acid (18:1) 38.2%, linoleic acid (18:2) 6.5%, linolenic acid (18:3) 2%, cholesterol 0.1%, and others 2%.

The Phospholipid Concentrate PC700™ phospholipid fraction (available from Fonterra Co-operative Group Limited, New Zealand) is produced by aqueous extraction (degumming) from the PC500™ phospholipid fraction. The PC700™ phospholipid fraction is a freeze dried milk phospholipid concentrate with a typical composition of 85% lipids, 10% ash, 2% lactose, and 2.5% moisture; a typical lipid composition of phosphatidyl serine 3%, phosphatidyl choline 31%, phosphatidyl ethanolamine 8.7%, and sphingomyelin 16.5%; and a typical fatty acid composition of myristic acid (14:0) 5.4%, palmitic acid (16:0) 20.9%, palmitoleic acid (16:1) 1.3%, margaric acid (17:0) 0.5%, stearic acid (18:0) 10.5%, oleic acid (18:1) 30.5%, linoleic acid (18:2) 4.3%, linolenic acid (18:3) 1.8%, and arachidonic acid 0.5%.

### Ganglioside fractions

Fractions 7 to 11 described in Table 7 above and used in the examples below were produced according to the methods described in published international patent application WO 2006/041316 (see examples 3 to 6). Fraction 12 was produced by supercritical carbon dioxide extraction of phospholipid fraction 10 in Table 7. Fraction 15 was produced by ethanol extraction of beta serum powder.

The G500™ and G600™ ganglioside fractions (available from Fonterra Co-operative Group Limited, New Zealand) are produced by ethanol extraction of beta serum powder. Beta serum is the liquid phase produced during AMF manufacture.

The G500™ ganglioside fraction is a spray dried milk ganglioside concentrate to which lactose and WPC (whey protein concentrate) has been added to improve powder flowability. The G500™ ganglioside fraction has a typical composition of lipids 34.0%, moisture 3.2%, ash 5.0%, and lactose 56.0%; a typical lipid composition of ganglioside GD3 0.6% and ganglioside GM3 0.5%; and a typical fatty acid composition of myristic acid (14:0) 5.6%, palmitic acid (16:0) 18.4%, palmitoleic acid (16:1) 1.2%, margaric acid (17:0) 0.5%, stearic acid (18:0) 14.9%, oleic acid (18:1) 31.0%, linoleic acid (18:2) 3.8%, linolenic acid (18:3) 1.5%, and arachidonic acid (20:4) 0.5%.

The Ganglioside G600™ ganglioside fraction is a spray dried milk ganglioside concentrate to which lactose has been added to improve powder flowability. The G600™ ganglioside fraction has a typical composition of lipids 30.0%, moisture 3.5%, ash 8.3%, and lactose 58.0%; a typical lipid composition of ganglioside GD3 1.4%, ganglioside GM3 0.3%, phosphatidyl serine 4.5%, phosphatidyl choline 5.1%, phosphatidyl ethanolamine 2.0%, and sphingomyelin 1.7%; and a typical fatty acid composition of myristic acid (14:0) 4.7%, palmitic acid (16:0) 16.4%, palmitoleic acid (16:1) 1.2%, margaric acid (17:0) 0.5%, stearic acid (18:0) 17.0%, oleic acid (18:1) 33.4%, linoleic acid (18:2) 4.2%, linolenic acid (18:3) 1.4%, and arachidonic acid (20:4) 0.6%.

### Lipid hydrolysates

Partial saponification was achieved by adding 800 µl of potassium hydroxide (1.5% in ethanol) to a 200 mg sample of a lipid preparation. The resulting solution was mixed for 10 minutes and neutralised to pH 7 with hydrochloric acid (10%). The solution was then flushed dry under nitrogen gas.

### Cell culture, virus propagation, and assay materials

All cell culture materials were purchased from Invitrogen. Rotavirus strain Wa (human) was a gift from John Taylor, Auckland University. HT29 cells were purchased from ATCC (HTB-38). Monosialoganglioside GM3 cat#1503 and disialoganglioside GD3 cat#1504 were purchased from Matreya (Pleasant Gap, PA, USA).

### Rotavirus propagation

The HT29 cell line was used to propagate the human rotavirus strain Wa (neuraminidase resistant) in the presence of 1µg/ml trypsin in Dulbecco's minimal essential medium (DMEM) supplemented with 10% foetal calf serum and penicillin/streptomycin (100µg/ml/100units/ml). Rotavirus was harvested by freeze thawing twice, spun at 750g to remove cell debris and stored at - 80°C. The rotavirus was assayed and found to contain 1 x 106 plaque forming units (pfu)/ml.

### Rotavirus infection assay

The infection assay used was based on a number of published assays (Scott et al 1979; Smith et al 1979; Guarino et al 1996; Superti et al 2001). Confluent HT29 cells grown in DMEM (seeded at 3x105/ml in 96 well plates) were washed twice in sterile PBS Doubling dilutions of each of the products to be screened (initial concentration 5mg/ml) were made in the plate in 50µl volumes of DMEM. Virus was added (104pfu/well) to all wells except the cells only control and plates were then incubated for two hours in 5% CO2 at 37°C. Other controls included virus plus cells, lipids only, pure GM3 and pure GD3 Following the two-hour incubation trypsin was added to all the wells containing virus at a concentration of 2µg/ml - this step cleaves VP4 to polypeptides VP8 and VP5 and is needed for the virion to penetrate into the cells'interior. Plates were incubated at 37°C in 5% CO2 for seven days and were checked daily for cytopathic effect in the virus only control wells. Media was removed and plates washed in sterile PBS, air-dried and fixed with methanol for one minute. Plates were air dried and then stained with 100µl/well of 1% crystal violet (351884W, BDH, England) for five minutes. Plates were washed four times with sterile water to remove excess stain 100µl of acetic acid per well was added to solubilise the stain and absorbance was read at a wavelength of 595 nm in a Biorad ELISA plate reader Each sample was assayed on a minimum of three separate occasions and the percentage of uninfected cells calculated.

### Statistics

Statistical analysis was performed using an unpaired Student t test (InStat for MacIntosh) to compare the effect of the lipid products derived from bovine milk with the virus only control at the lowest dilution of the product.

### EXAMPLE 1 - Dairy lipids inhibit rotavirus infection in vitro

Sixteen lipid test materials were screened for their ability to prevent rotavirus infection of cells. None of the lipid test materials or the comparative materials were toxic to the HT29 cells. Gangliosides GD3 and GM3 both showed activity over a range of concentrations (Table 8). Of the sixteen lipid test materials, fourteen showed activity in preventing virus infection of the cells over a range of concentrations (Table 9)

**Table 8: Percentage of HT29 cells uninfected by rotavirus in the presence of GD3 and GM3 at various concentrations**

| **Sample** | **250 µg/ml** | **125 µg/ml** | **62.5 µg/ml** | **31.25 µg/ml** | **15.625 µg/ml** | **7.8125 µg/ml** |
|---|---|---|---|---|---|---|
| **Ganglioside Material** | | | | | | |
| **GD3** | 57 +/- 1 *** | ND | 59 +/- 1.4 *** | 40 +/- 2.5 *** | 31 +/- 1.5 | ND |
| **GM3** | ND | 49 +/- 8 | ND | 43 +/- 10** | 33 +/- 2.5 | 34 +/- 1.5 |

| | | | | | | |
|---|---|---|---|---|---|---|
| *significant at p<0.01 compared to virus-only control; **significant at p<0.005; ***significant at p<0.001; samples with no p value are not significantly different from the virus-only control. ND = not done. | | | | | | |

**Table 9: Percentage of HT29 cells uninfected by rotavirus in the presence of various lipid fractions from bovine milk at various concentrations**

| **Sample** | **5 mg/ml** | **2.5 mg/ml** | **1.25 mg/ml** | **0.625 mg/ml** | **0.3125 mg/ml** |
|---|---|---|---|---|---|
| **Comparative Materials** | | | | | |
| **Lactoferrin** | 67 +/- 4*** | 87 +/- 4*** | 73 +/- 12*** | 65 +/- 8*** | 60 +/- 8** |
| **Sialyl oligolac** | 20 +/- 8 | 24 +/- 2 | 37 +/- 4 | 35 +/- 8 | 35 +/- 0.7 |
| **MFGM** | 17 +/- 1.5 | 18 +/- 3.5 | 21 +/- 6 | 23 +/- 7 | 24 +/- 8 |
| **MFGM protein** | 51 +/- 1.4 | 35.5 +/- 2 | 26 +/- 3 | 31 +/- 4 | 35 +/- 1.4 |

| **Test Materials** | | | | | |
|---|---|---|---|---|---|
| **1. Milkfat** | | | | | |
| **AMF** | 44 +/- 19 | 26 +/- 3 | 35 +/ 1* | **41** +/- 2*** | 42 +/- 5** |

| **2. Milkfat Fractions** | | | | | |
|---|---|---|---|---|---|
| **SH** | 23 +/- 9 | 40 +/- 10 | 45 +/- 10 | 40 +/- 5 | 42 +/- 8* |
| **SSH** | 28 +/- 5 | 34 +/- 6 | 47 +/- 17* | 41 +/- 11* | 39 +/- 11* |
| **SSS** | 31 +/- 4 | 38 +/- 5** | 41 +/- 2*** | 44 +/- 2*** | 37 +/- 5** |

| **3. High CLA milkfat** | | | | | |
|---|---|---|---|---|---|
| **Batch 1 (2002)** | 27 +/- 13 | 63 +/- 35** | 44 +/- 13** | 44 +/- 15** | 49 +/- 13*** |
| **Batch 2 (2005)** | 30 +/- 9 | 46 +/- 9*** | 45 +/- 7*** | 47 +/- 9*** | 42 +/- 8 *** |

| **4. Beta serum** | | | | | |
|---|---|---|---|---|---|
| **Batch 1** | 58 +/- 4*** | 56 +/- 7*** | 56 +/- 10*** | 46 +/- 11*** | 44 +/- 7*** |

| **5. Phospholipid fractions** | | | | | |
|---|---|---|---|---|---|
| **PC500** | 41 +/- 14* | 56 +/- 13 *** | 46 +/- 9*** | 43 +/- 9*** | 41 +/- 8** |
| **PC600** | 41 +/- 18* | 55 +/- 16 *** | 42 +/- 13* | 45 +/- 18* | 46 +/- 13** |
| **PC700** | 50 +/- 45 | 61 +/- 17 *** | 62 +/- 25*** | 51 +/- 10 *** | 44 +/- 9*** |

| **6. Ganglioside fractions** | | | | | |
|---|---|---|---|---|---|
| **Sample** | **5 mg/ml** | **2.5 mg/ml** | **1.25 mg/ml** | **0.625 mg/ml** | **0.3125 mg/ml** |
| **Fraction 8** | 61 +/- 2*** | 54 +/- 3** | 36 +/- 1.1* | 23 +/- 3 | 23 +/- 12 |
| **Fraction 9** | 40 +/- 1* | 35 +/- 7 | 47 +/- 15 | 36 +/- 2 | 30 +/- 2 |
| **Fraction 11** | 45 +/- 3* | 48 +/- 1* | 36 +/- 0.7 | 25 +/- 1 | 17 +/- 2 |
| **G500** | 60 +/- 12*** | 66 +/- 5*** | 56 +/- 7*** | 52 +/- 9*** | 44 +/- 4*** |
| **G600** | 44 +/- 17* | 26 +/- 14 | 35 +/- 4 | 41 +/- 4*** | 42 +/- 4*** |

| **7. Hydrolysed ganglioside fractions** | | | | | |
|---|---|---|---|---|---|
| **G500** | 97 +/- 21*** | 20 +/- 5 | 33 +/- 2 | 22 +/- 6 | 22 +/- 6 |
| **G600** | 25 +/- 0.7 | 18 +/- 3 | 21 +/- 4 | 22 +/- 6 | 22 +/- 5 |

| | | | | | |
|---|---|---|---|---|---|
| *significant at p<0.01 compared to virus-only control; ** significant at p<0.005; *** significant at p<0.001; samples with no p value are not significantly different from the virus-only control. | | | | | |

Five test materials significantly reduced the rate of infection at a concentration of 0.3125 mg/ml at p<0.001 (Table 9). These test materials were CLA-enriched milk fat, beta serum, the PC700™ phospholipid fraction, and the ganglioside fractions. Four test materials significantly reduced the rate of infection at a concentration of 0.3125 mg/ml at p<0.005 These test materials were AMF, a soft milkfat fraction (SSS), and the PC500™ and PC600™ phospholipid factions. Three test materials significantly reduced the rate of infection at a concentration of 0.3125 mg/ml at p<0 01 These test materials were other the SH and SSH milkfat fractions, and a hydrolysed ganglioside fraction The remaining products were not significantly different from the virus control. Purified samples of GM3 and GD3 protected cells from virus infection at concentrations as low as 31.25 µg/ml (43+/-10% and 40+/-3% respectively; p<0.005). On a ganglioside-concentration basis, the G600™ fraction is ten times more effective than pure GD3 at the lowest G600™ concentration

### EXAMPLE 2 - Dairy lipids can treat or prevent diarrhea caused by rotavirus infection

A baby rat animal model for human rotavirus infection can be used to determine efficacy of the products in vivo (see for example, Ciarlet et al 2006).

Five-day-old rat pups in groups of ten were fed four different lipid test materials at a concentration of 3 g/kg body weight by delivery into the oral cavity in a volume of 100 µl. All other feeding came from the lactating mother rat. On Days 1 and 2, the rat pups were given two 100 µl doses (morning and evening) of human rotavirus strain Wa. Negative control rats were inoculated with 100 µl phosphate-buffered saline (PBS) but were not infected with virus. Positive control rats were inoculated with 100 µl PBS and were subsequently infected with virus The experiment was run for 10 days following inoculation with rotavirus. The experiments were run in duplicate

The rats were weighed daily in order to determine the infection rate. Collection of individual faecal samples into pre-weighed containers and determination of diarrhea were performed once a day Diarrhea was scored daily from 1 to 4 based on colour, consistency and amount of stool as follows.
1. Normal stools - normal colour (brown) and consistency.
2. Mild diarrhea - abnormal colour (green or yellow green) but normal consistency.
3. Moderate diarrhea - normal colour (brown) but watery consistency.
4. Severe diarrhea - abnormal colour and watery consistency.

Faecal samples were processed as a 10% solution in cold (4°C) PBS containing penicillin (200 U/ml), streptomycin (200 µl/ml) and gentamicin (2 mg/ml), and 10 µl aliquots were assayed in duplicate in cell culture as described above to determine the levels of infectious rotavirus These levels were expressed as the percentage of uninfected cells in the in vitro assay: % uninfected cells = (OD of test sample) * 100 / (OD of negative control), where OD is the optical density of the solution in the well.

**Table 10: Severity of diarrhea in rats fed high CLA milk fat**

| | **Control - no virus** | | **Infected** | | **High CLA milkfat** | |
|---|---|---|---|---|---|---|
| **Days post-infection** | **Number** | **Severity** | **Number** | **Severity** | **Number** | **Severity** |
| 3 | 4 | 1 | 7 | 1 | 6 | 1 |
| 4 | 5 | 1 | 8 | 3 | 5 | 1 |
| 5 | 5 | 1 | 5 | 2 | 5 | 2 |
| 6 | 5 | 1 | 8 | 3 | 3 | 2 |
| 7 | 5 | 1 | 4 | 2 | 2 | 2 |
| 8 | 4 | 1 | 5 | 2 | 2 | 2 |

**Table 11: Severity of diarrhea in rats fed the G600™ milk fat fraction**

| | **Control - no virus** | | **Infected** | | **G60I™** | |
|---|---|---|---|---|---|---|
| **Days post-infection** | **Number** | **Severity** | **Number** | **Severity** | **Number** | **Severity** |
| 3 | 5 | 1 | 6 | 2 | 6 | 1 |
| 4 | 5 | 1 | 4 | 2 | 4 | 1 |
| 5 | 5 | 1 | 4 | 2 | 4 | 1 |
| 6 | 6 | 1 | 7 | 3 | 7 | 1 |
| 7 | 4 | 1 | 4 | 3 | 4 | 1 |
| 8 | 1 | 1 | 4 | 2 | 4 | 1 |

**Table 12: Severity of diarrhea in rats fed a fraction of beta serum**

| | **Control - no vitus** | | **Infected** | | **Fraction 10** | | **Fraction 8** | |
|---|---|---|---|---|---|---|---|---|
| **Days post-infection** | **#** | **Severity** | **#** | **Severity** | **#** | **Severity** | **#** | **Severity** |
| 3 | 0 | | 10 | 2 | 6 | 2 | 5 | 2 |
| 4 | 0 | | 7 | 3 | 9 | 1 | 8 | 2 |
| 5 | 2 | 1 | 2 | 2 | 4 | 1 | 4 | 2 |
| 6 | 1 | 1 | 4 | 3 | 7 | 1 | 6 | 2 |
| 7 | 2 | 1 | 3 | 2 | 6 | 1 | 7 | 2 |
| 8 | 1 | 1 | 4 | 2 | 9 | 1 | 8 | 2 |
| 9 | 10 | 1 | 10 | 2 | 10 | 1 | 10 | 2 |

Faecal samples were assayed in the in vitro rotaviral assay to determine the percentage of uninfected cells. The differences between treatment and infected-control for rats fed high CLA milk fat (Figure 1A) were significant on days 1 (P<0.001), 3 (P<0.025), 7 (P<0.05) and 8 (P<0.025). The differences between treatment and infected-control for rats fed the G600™ fraction (Figure 1B) were significant on days 3 (P<0.001), 4 (P<0.001), 6 (P=0.001), 7 (P<0.001) and 8 (P=0.001).

### INDUSTRIAL APPLICATION

The present invention has utility in treating or preventing rotavirus infection. The described compositions may be employed as foods, drinks, food additives, drink additives, dietary supplements, nutritional products, medical foods, nutraceuticals, medicaments or pharmaceuticals.

Those persons skilled in the art will understand that the above description is provided by way of illustration only and that the invention is not limited thereto.

### REFERENCES

Ainscough, EW, Brodie A, M.; Plowman J E. The chromium, manganese, cobalt and copper complexes of human lactoferrin. Inorganica Chimica Acta 1979,33 (2) 149-53.
Astaire J. C., Ward R., German J. B., and Jimenez-Flores R. (2003) Concentration of Polar MFGM Lipids from Buttermilk by Microfiltration and Supercritical Fluid Extraction J. Dairy Sci. 86, 2297-2307
Bojsen A, J Buesa, R Montava, A S Kvistgaard, M B Kongsbak, T E Peterson, C W Heegaard and J T Rasmussen 2007 Inhibitory Activities of Bovine macromolecular Whey proteins on Rotavirus Infections In Vitro and In Vivo J Dairy Sci 90:66-74.
Bylund, G. (Ed.) Dairy processing handbook. 1995 Tetra Pak Processing Systems AB, S-221 86 Lund, Sweden.
Chin, S.F., Liu, W., Storkson, J.M., Ha, Y.L. and Pariza, M.W. Dietary sources of conjugated dienoic isomers of linoleic acid, a newly recognised class of anticarcinogens, Journal of Food composition and Analysis, 5, 185-197, 1992.
Max Ciarlet, Margaret E Conner, Milton J Finegold and Mary K Estes 2002. Group A Rotavirus Infection and age-Dependent Diarrheal Disease in Rats: a New Animal Model to Study the Pathophysiology of Rotavirus Infection. Journal of Virology 76 (1): 41-57
Scott M. Clark, Bill B. Barnett and Rex S. Spendlove 1979. Production of high-titer bovine rotavirus with trypsin. Journal of Clinical Microbiology Mar: 413-417.
Delorme C, Brüssow H, Sidoti J, Roche N, Karlsson KA, Neeser JR, Teneberg S., Glycosphingolipid binding specificities of rotavirus: identification of a sialic acid-binding epitope. J Virol. 2001 Mar;75(5):2276-87.
Fox PF, McSweeney PLH (eds), Advanced Dairy Chemistry, Volume 2 - Lipids, 3rd Ed, Springer Science + Business Media, Inc., 2006.
Freireich EJ, Gehan EA, Rall DP, Schmidt LH, Skipper HE (1966) Quantitative comparison of toxicity to anticancer agents in mouse, rat, hamster, dog, monkey and man. Cancer Chemother Rep 50: 219-244.
Guarino A, Casola A, Bruzzese E, Saini M, Nitsch L, Rubino A. 1996. Human serum immunoglobulin counteracts rotaviral infection in Caco-2 cells. Pediatric Research 40(6): 887-7.
Guo CT, Nakagomi O, Mochizuki M, Ishida H, Kiso M, Ohta Y, Suzuki T, Miyamoto D, Hidari KI, Suzuki Y., Ganglioside GM(1a) on the cell surface is involved in the infection by human rotavirus KUN and MO strains. J Biochem (Tokyo). 1999 Oct;126(4):683-8.
Illingworth, D., Fractionation of fats. In Physical Properties of Lipids (Marangoni A G & Narine S S, Eds), pp. 411-448. Marcel Dekker, New York (2002).
Kanno C & Dong-Hyun K (1990). A simple procedure for the preparation of bovine milk fat globule membrane and a comparison of its composition, enzymatic activity, and electrophoretic properties with these prepared by other methods. Agric. Biol. Chem., 54(11):2845-2854.
Kanno C, Shimizu M & Yamachi K (1975). Isolation and physiochemical properties of a soluble glycoprotein fraction of milk fat globule membrane. Agric. Biol. Chem., 39(9):1835-1842.
Takahashi Kazuo, Katzutaka Ohashi, Yurika Abe, Shuichi Mori, Koki Taniguchi, Takusaburo Ebina, Osamu Nakagomi, Masaki Terada and Shiro Shigeta 2002. Protective Efficacy of a Sulfated Sialyl Lipid (NMSO3) against Human Rotavirus-Induced Diarrhea in a Mouse Model. Antimicrobial Agents and Chemotherapy 46: 420 -424.
Kvistgaard AS, Pallesen LT, Arias CF, Lopez S, Petersen TE, Heegaard CW, Rasmussen JT., Inhibitory effects of human and bovine milk constituents on rotavirus infections. J Dairy Sci. (2004) 87(12):4088-96.
Méndez E, López S, Cuadras MA, Romero P, Arias CF., Entry of rotaviruses is a multistep process. Virology. 1999 Oct 25;263(2):450-9.
Parashar, UD, EG Hummelman, JS Bresee, MA Miller and RI Glass 2003. Global illness and deaths caused by rotavirus disease in children Emerg Infect Dis 9:565-572
Isa Pavel, Carlos F Arias, Susana Lopez 2006. Role of sialic acids in rotavirus infection. Glycoconj J 23:27-37
Peterson JA, Scallan CD, Ceriaii RL, Hamosh M 2001. Structural and functional aspects of three major glycoproteins of the human milk fat globular membrane. Adv Exp Med Biol 501:179-87
Pruthi T D, Narayanan K M & Bhaleerao V R (1970). The role of milk phospholipids in the autoxidation of butterfat - I. Indian Journal of Dairy Science, 23:248-251.
Rombaut R, Camp JV, Dewettinck K., Analysis of phospho- and sphingolipids in dairy products by a new HPLC method. J Dairy Sci. (2005) 88(2):482-8.
Rombaut R, Dejonckheere V, Dewettinck K., Microfiltration of butter serum upon casein micelle destabilization. J Dairy Sci. (2006)(a) 89(6):1915-25.
Rombaut R., Van Camp J. & Dewettinck K., Phospho- and sphingolipid distribution during processing of milk, butter and whey, International Journal of Food Science & Technology, (2006)(b) 41(4):435-443.
Sambrook, J.; Fritsch, E.F.; Maniatis, T. (1989). Molecular Cloning: A Laboratory Manual. Cold Spring Harbour Lab Press, Cold Spring Harbour, New York.
Eric M. Smith, Mary Kolb Estes, David Y. Graham and Charles P. Gerba 1979. A plaque assay for the simian rotavirus SA11. Journal of General Virology 43: 513-519.
Fabiana Superti, Rosa Siciliano, Barbara Rega, Francesco Giansanti, Piera Valenti, Giovanni Antonini 2001. Involvement of bovine lactoferrin metal saturation, sialic acid and protein fragments in the inhibition of rotavirus infection. Biochimica et Biophysica Acta 1528:107-115.
van Veen HA, Geerts ME, van Berkel PH, Nuijens JH. The role of N-linked glycosylation in the protection of human and bovine lactoferrin against tryptic proteolysis. Eur. J. Biochem. (2004) 271(4): 678-684.
Wolber FM, A M Broomfield, L Fray, M L Cross and D Dey 2005. Supplemental dietary Whey Protein Concentrate Reduces Rotavirus-Induced Symptoms in Suckling Mice. Journal of Nutrition 135: 1470 1474
Yung A, McDonald M, Spelman D, Street A, Johnson P, Sorrell T, McCormack J, "Infectious Diseases: A Clinical Approach", Second edition 2005. Ed. (IP Communications PTY Ltd., Victoria, Australia). See page 129, Chapter 11, "Diarrhoea and Vomiting".

## Claims

1. An agent comprising
(a) one or more bovine milk fat compositions comprising about 5 to about 95% w/w lipid, about 0 to about 75% w/w protein, about 5 to about 85% w/w phospholipid and about 0 to about 5% w/w ganglioside selected from
(i) one or more phospholipid-enriched fractions of milk fat,
(ii) one or more ganglioside-enriched fractions of milk fat,
(iii) one or more hydrolysates of any one or more of (i) to (ii), and
(iv) a combination of any two or more of (i) to (iii);
for the use in treating or preventing rotavirus infection in a subject, wherein the rotavirus is a neuraminidase-resistant rotavirus.

2. The agent for the use of claim 1 wherein the rotavirus is a human rotavirus.

3. The agent for the use of claim 1 or 2 wherein the subject is a human, a child, an infant, an immunocompromised child, an immunocompromised infant, an adult, an adult over the age of 55, an immunocompromised adult, or an immunocompromised adult over the age of 55.

4. The agent for the use of any one of claims 1 to 3 for treating or preventing diarrheagenic rotavirus infection or for treating or preventing diarrhea caused by rotavirus infection.

5. The agent for the use of any one of claims 1 to 4 wherein the agent further comprises anhydrous milk fat (AMF) comprising about 40 to about 100% lipid.

6. The agent for the use of any one of claims 1 to 5 comprising one or more phospholipid-enriched fractions of bovine milk fat or one or more ganglioside-enriched fractions of bovine milk fat or a combination thereof, for treating or preventing rotavirus infection in a subject.

7. The agent for the use of any one of claims 1 to 6 wherein the phospholipid-enriched fraction is selected from buttermilk, one or more buttermilk fractions, butter serum, one or more butter serum fractions, beta serum, one or more beta serum fractions, one or more sphingolipid fractions, one or more milk fat globule membrane lipid fractions, one or more phospholipid fractions, one or more complex lipid fractions, and any combination of any two or more thereof, wherein beta serum is separated from a dairy stream containing greater than 60% fat, the dairy stream being cream that has been through phase inversion from an oil-in-water to a water-in-oil emulsion during production of butter oil or anhydrous milk fat from cream.

8. The agent for the use of any one of claims 1 to 6 wherein the ganglioside-enriched fraction is selected from buttermilk, one or more buttermilk fractions, butter serum, one or more butter serum fractions, beta serum, one or more beta serum fractions, one or more GD3-enriched fractions of beta serum, one or more GM3-enriched fractions of beta serum, one or more GD3- and GM3-enriched fractions of beta serum, and any combination of any two or more thereof, wherein beta serum is separated from a dairy stream containing greater than 60% fat, the dairy stream being cream that has been through phase inversion from an oil-in-water to a water-in-oil emulsion during production of butter oil or anhydrous milk fat from cream.

9. The agent for the use of any one of claims 1 to 4 further comprising anhydrous bovine milk fat (AMF), one or more AMF fractions, or a combination thereof, for treating or preventing rotavirus infection in a subject.

10. The agent for the use of any one of claims 1 to 4 wherein the agent further comprises AMF fraction selected from one or more hard milk fat fractions, one or more soft milk fat fractions, a combination of hard milk fat fractions, a combination of soft milk fat fractions, a combination of hard milk fat fractions and soft milk fat fractions, and any combination of any two or more thereof.

11. The agent for the use of claims 10 wherein the AMF comprises about 98 to about 100% milk fat.

12. The agent for the use of any one of claims 6 to 8 wherein the fraction comprises
(a) about 15 to about 95% w/w lipid and about 0 to about 75% w/w protein, or
(b) about 15 to about 95% w/w lipid, about 0 to about 65% w/w protein, about 5 to about 70% w/w phospholipids and about 0 to about 2.5% w/w ganglioside.

13. The agent for the use of any one of claims 6 to 8 wherein the fraction comprises
(a) about 25 to about 35% w/w protein, about 12 to about 25% w/w lipid, about 5 to about 15% w/w phospholipid, about 5 to about 15% w/w MFGM protein, and about 0.2 to about 0.9% w/w ganglioside, or
(b) about 40 to about 60% w/w protein, about 25 to about 45% w/w lipid, about 10 to about 25% w/w phospholipid, about 5 to about 20% w/w MFGM protein, and about 0.5 to about 2.0% w/w ganglioside, or
(c) about 50 to about 70% w/w protein, about 12 to about 32% w/w lipid, about 5 to about 25% w/w phospholipid, about 2 to about 8 % w/w phosphatidylcholine, about 2 to about 10% w/w phosphatidylethanolamine, about 2 to about 8% w/w sphingomyelin, and about 1 to about 3% w/w phosphatidylserine, about 10 to about 20% w/w MFGM protein, and about 0.5 to about 2.5% w/w ganglioside, or
(d) about 0 to about 10% w/w protein, about 85 to about 97% w/w lipid, about 25 to about 35% w/w phospholipid, about 5 to about 10 % w/w phosphatidylcholine, about 7 to about 13% w/w phosphatidylethanolamine, about 4 to about 9% w/w sphingomyelin, about 2 to about 5% w/w phosphatidylserine, about 1 to about 3% w/w phosphatidylinositol, about 0 to about 5 % w/w MFGM protein, and about 1 to about 3% w/w gangliosides, or
(e) about 10 to about 15% w/w protein, about 80 to about 95% w/w lipid, about 60 to about 80% w/w phospholipid, about 10 to about 20 % w/w phosphatidylcholine, about 18 to about 28% w/w phosphatidylethanolamine, about 10 to about 20% w/w sphingomyelin, about 4 to about 12% w/w phosphatidylserine, about 2 to about 10% w/w phosphatidylinositol, about 0 to about 5% w/w MFGM protein, and about 1 to about 5% w/w gangliosides, or
(f) about 75 to about 99% w/w lipid, about 15 to 35% w/w phospholipid, about 5 to about 15% w/w phosphatidylcholine, about 5 to about 15% w/w phosphatidylethanolamine, about 4 to about 15% w/w sphingomyelin, about 0.1 to about 2% w/w phosphatidylserine, and about 0.1 to about 2% w/w phosphatidylinositol, or
(g) about 75 to about 95% w/w lipid, about 50 to about 90% w/w phospholipid, about 10 to about 45% w/w phosphatidylcholine, about 12 to about 25% w/w phosphatidylethanolamine, about 12 to about 25% w/w sphingomyelin, about 1 to about 6% w/w phosphatidylserine, and about 0.5 to 4% w/w phosphatidylinositol or
(h) about 80 to about 90% w/w lipid, about 65 to about 75% w/w phospholipid, about 10 to about 30% w/w phosphatidylcholine, about 12 to about 22% w/w phosphatidylethanolamine, about 12 to about 22% w/w sphingomyelin, and about 1 to about 3% w/w phosphatidylserine, or
(i) about 25 to about 45% w/w lipid, about 10 to about 30% w/w phospholipids, about 2 to about 5% w/w phosphatidylcholine, about 3 to about 7% w/w phosphatidylethanolamine, about 2 to about 5% w/w sphingomyelin, about 2 to about 12% w/w phosphatidylserine, about 1 to about 5% w/w phosphatidylinositol, and about 0.2 to about 1% w/w ganglioside, or
(j) about 20 to about 40% w/w lipid, about 5 to about 30% w/w phospholipids, about 1 to about 5% w/w phosphatidylcholine, about 2 to about 8% w/w phosphatidylethanolamine, about 0.5 to about 5% w/w sphingomyelin, about 1 to about 10% w/w phosphatidylserine, about 1 to about 6% w/w phosphatidylinositol, and about 0.8 to about 3.5% w/w ganglioside.

14. The agent for the use of any one of claims 1 to 13 wherein the agent further comprises lactoferrin, iron-lactoferrin, a lactoferrin fragment, or an iron-lactoferrin fragment, or a combination of any two or more thereof.

15. The use of the agent as set forth in any one of the preceding claims, the agent comprising (a) one or more bovine milk fat compositions comprising about 5 to about 95% w/w lipid, about 0 to about 75% w/w protein, about 5 to about 85% w/w phospholipid and about 0 to about 5% w/w ganglioside selected from
i. one or more phospholipid-enriched fractions of milk fat,
ii. one or more ganglioside-enriched fractions of milk fat,
iii. one or more hydrolysates of any one or more of (i) to (ii), and
a combination of any two or more of (i) to (iii) for the manufacture of a medicament for the use in treating or preventing rotavirus infection in a subject, wherein the rotavirus is a neuraminidase-resistant rotavirus.

## Patentansprüche

1. Mittel, umfassend
(a) eine oder mehrere Kuhmilchfettzusammensetzungen umfassend etwa 5 bis etwa 95 Gewichtsprozent Lipid, etwa 0 bis etwa 75 Gewichtsprozent Protein, etwa 5 bis etwa 85 Gewichtsprozent Phospholipid und etwa 0 bis etwa 5 Gewichtsprozent Gangliosid ausgewählt aus
(i) einer oder mehreren phospholipidangereicherten Milchfettfraktionen,
(ii) einer oder mehreren gangliosidangereicherten Milchfettfraktionen,
(iii) einer oder mehreren Hydrolysaten von einem oder mehreren von (i) bis (ii), sowie
(iv) einer Kombination von zweien oder mehreren von (i) bis (iii);
zur Verwendung bei der Behandlung oder Verhinderung von Rotavirusinfektionen in einem Individuum, wobei das Rotavirus ein Neuraminidase-resistentes Rotavirus ist.

2. Mittel zur Verwendung nach Anspruch 1, wobei das Rotavirus ein menschliches Rotavirus ist.

3. Mittel zur Verwendung nach Anspruch 1 oder 2, wobei das Individuum ein Mensch, ein Kind, ein Kleinkind, ein immunkompromittiertes Kind, ein immunkompromittiertes Kleinkind, ein Erwachsener, ein Erwachsener im Alter von über 55, ein immunkompromittierter Erwachsener, oder ein immunkompromittierter Erwachsener im Alter von über 55 ist.

4. Mittel zur Verwendung nach einem der Ansprüche 1 bis 3 zur Behandlung oder Verhinderung diarrhogener Rotavirusinfektionen oder zur Behandlung oder Verhinderung von durch Rotavirusinfektionen verursachter Diarrhö.

5. Mittel zur Verwendung nach einem der Ansprüche 1 bis 4, wobei das Mittel weiter wasserfreies Milchfett (AMF) umfassend etwa 40 bis etwa 100 % Lipid enthält.

6. Mittel zur Verwendung nach einem der Ansprüche 1 bis 5, umfassend eine oder mehrere phospholipidangereicherte Kuhmilchfettfraktionen oder eine oder mehrere gangliosidangereicherte Kuhmilchfettfraktionen oder eine Kombination davon, zur Behandlung oder Verhinderung von Rotavirusinfektionen in einem Individuum.

7. Mittel zur Verwendung nach einem der Ansprüche 1 bis 6, wobei die phospholipidangereicherte Fraktion ausgewählt ist aus Buttermilch, einer oder mehreren Buttermilchfraktionen, Butterserum, einer oder mehreren Butterserumfraktionen, Betaserum, einer oder mehreren Betaserumfraktionen, einer oder mehreren Sphingolipidfraktionen, einer oder mehreren Milchfettglobuli-Membranlipidfraktionen, einer oder mehreren Phospholipidfraktionen, einer oder mehreren komplexen Lipidfraktionen, und einer Kombination von zweien oder mehreren davon, wobei Betaserum von einem Milchstrom enthaltend mehr als 60 % Fett getrennt wird, wobei es sich bei dem Milchstrom um Sahne handelt, die während der Herstellung von Butteröl oder wasserfreiem Milchfett aus Sahne eine Phasenumkehr von einer Öl-in-Wasser- zu einer Wasser-in-Öl-Emulsion durchlaufen hat.

8. Mittel zur Verwendung nach einem der Ansprüche 1 bis 6, wobei die gangliosidangereicherte Fraktion ausgewählt ist aus Buttermilch, einer oder mehreren Buttermilchfraktionen, Butterserum, einer oder mehreren Butterserumfraktionen, Betaserum, einer oder mehreren Betaserumfraktionen, einer oder mehreren GD3-angereicherten Betaserumfraktionen, einer oder mehreren GM3-angereicherten Betaserumfraktionen, einer oder mehreren GD3- und GM3-angereicherten Betaserumfraktionen, und einer Kombination von zweien oder mehreren davon, wobei Betaserum von einem Milchstrom enthaltend mehr als 60 % Fett getrennt wird, wobei es sich bei dem Milchstrom um Sahne handelt, die während der Herstellung von Butteröl oder wasserfreiem Milchfett aus Sahne eine Phasenumkehr von einer Öl-in-Wasser- zu einer Wasser-in-Öl-Emulsion durchlaufen hat.

9. Mittel zur Verwendung nach einem der Ansprüche 1 bis 4, ferner umfassend wasserfreies Kuhmilchfett (AMF), eine oder mehrere AMF-Fraktionen, oder eine Kombination davon, zur Behandlung oder Verhinderung von Rotavirusinfektionen in einem Individuum.

10. Mittel zur Verwendung nach einem der Ansprüche 1 bis 4, wobei das Mittel ferner eine AMF-Fraktion umfasst, die ausgewählt ist aus einer oder mehreren Milchhartfettfraktionen, einer oder mehreren Milchweichfettfraktionen, einer Kombination aus Milchhartfettfraktionen, einer Kombination aus Milchweichfettfraktionen, einer Kombination aus Milchhartfettfraktionen und Milchweichfettfraktionen, und einer Kombination von zweien oder mehreren davon.

11. Mittel zur Verwendung nach Anspruch 10, wobei das AMF etwa 98 bis etwa 100 % Milchfett umfasst.

12. Mittel zur Verwendung nach einem der Ansprüche 6 bis 8, wobei die Fraktion umfasst
(a) etwa 15 bis etwa 95 Gewichtsprozent Lipid und etwa 0 bis etwa 75 Gewichtsprozent Protein, oder
(b) etwa 15 bis etwa 95 Gewichtsprozent Lipid, etwa 0 bis etwa 65 Gewichtsprozent Protein, etwa 5 bis etwa 70 Gewichtsprozent Phospholipide und etwa 0 bis etwa 2,5 Gewichtsprozent Gangliosid.

13. Mittel zur Verwendung nach einem der Ansprüche 6 bis 8, wobei die Fraktion umfasst:
(a) etwa 25 bis etwa 35 Gewichtsprozent Protein, etwa 12 bis etwa 25 Gewichtsprozent Lipid, etwa 5 bis etwa 15 Gewichtsprozent Phospholipid, etwa 5 bis etwa 15 Gewichtsprozent MFGM-Protein, und etwa 0,2 bis etwa 0,9 Gewichtsprozent Gangliosid, oder
(b) etwa 40 bis etwa 60 Gewichtsprozent Protein, etwa 25 bis etwa 45 Gewichtsprozent Lipid, etwa 10 bis etwa 25 Gewichtsprozent Phospholipid, etwa 5 bis etwa 20 Gewichtsprozent MFGM-Protein, und etwa 0,5 bis etwa 2,0 Gewichtsprozent Gangliosid, oder
(c) etwa 50 bis etwa 70 Gewichtsprozent Protein, etwa 12 bis etwa 32 Gewichtsprozent Lipid, etwa 5 bis etwa 25 Gewichtsprozent Phospholipid, etwa 2 bis etwa 8 Gewichtsprozent Phosphatidylcholin, etwa 2 bis etwa 10 Gewichtsprozent Phosphatidylethanolamin, etwa 2 bis etwa 8 Gewichtsprozent Sphingomyelin, und etwa 1 bis etwa 3 Gewichtsprozent Phosphatidylserin, etwa 10 bis etwa 20 Gewichtsprozent MFGM-Protein, und etwa 0,5 bis etwa 2,5 Gewichtsprozent Gangliosid, oder
(d) etwa 0 bis etwa 10 Gewichtsprozent Protein, etwa 85 bis etwa 97 Gewichtsprozent Lipid, etwa 25 bis etwa 35 Gewichtsprozent Phospholipid, etwa 5 bis etwa 10 Gewichtsprozent Phosphatidylcholin, etwa 7 bis etwa 13 Gewichtsprozent Phosphatidylethanolamin, etwa 4 bis etwa 9 Gewichtsprozent Sphingomyelin, etwa 2 bis etwa 5 Gewichtsprozent Phosphatidylserin, etwa 1 bis etwa 3 Gewichtsprozent Phosphatidylinositol, etwa 0 bis etwa 5 Gewichtsprozent MFGM-Protein, und etwa 1 bis etwa 3 Gewichtsprozent Ganglioside, oder
(e) etwa 10 bis etwa 15 Gewichtsprozent Protein, etwa 80 bis etwa 95 Gewichtsprozent Lipid, etwa 60 bis etwa 80 Gewichtsprozent Phospholipid, etwa 10 bis etwa 20 Gewichtsprozent Phosphatidylcholin, etwa 18 bis etwa 28 Gewichtsprozent Phosphatidylethanolamin, etwa 10 bis etwa 20 Gewichtsprozent Sphingomyelin, etwa 4 bis etwa 12 Gewichtsprozent Phosphatidylserin, etwa 2 bis etwa 10 Gewichtsprozent Phosphatidylinositol, etwa 0 bis etwa 5 Gewichtsprozent MFGM-Protein, und etwa 1 bis etwa 5 Gewichtsprozent Ganglioside, oder
(f) etwa 75 bis etwa 99 Gewichtsprozent Lipid, etwa 15 bis etwa 35 Gewichtsprozent Phospholipid, etwa 5 bis etwa 15 Gewichtsprozent Phosphatidylcholin, etwa 5 bis etwa 15 Gewichtsprozent Phosphatidylethanolamin, etwa 4 bis etwa 15 Gewichtsprozent Sphingomyelin, etwa 0,1 bis etwa 2 Gewichtsprozent Phosphatidylserin, und etwa 0,1 bis etwa 2 Gewichtsprozent Phosphatidylinositol, oder
(g) etwa 75 bis etwa 95 Gewichtsprozent Lipid, etwa 50 bis etwa 90 Gewichtsprozent Phospholipid, etwa 10 bis etwa 45 Gewichtsprozent Phosphatidylcholin, etwa 12 bis etwa 25 Gewichtsprozent Phosphatidylethanolamin, etwa 12 bis etwa 25 Gewichtsprozent Sphingomyelin, etwa 1 bis etwa 6 Gewichtsprozent Phosphatidylserin, und etwa 0,5 bis etwa 4 Gewichtsprozent Phosphatidylinositol, oder
(h) etwa 80 bis etwa 90 Gewichtsprozent Lipid, etwa 65 bis etwa 75 Gewichtsprozent Phospholipid, etwa 10 bis etwa 30 Gewichtsprozent Phosphatidylcholin, etwa 12 bis etwa 22 Gewichtsprozent Phosphatidylethanolamin, etwa 12 bis etwa 22 Gewichtsprozent Sphingomyelin, und etwa 1 bis etwa 3 Gewichtsprozent Phosphatidylserin, oder
(i) etwa 25 bis etwa 45 Gewichtsprozent Lipid, etwa 10 bis etwa 30 Gewichtsprozent Phospholipide, etwa 2 bis etwa 5 Gewichtsprozent Phosphatidylcholin, etwa 3 bis etwa 7 Gewichtsprozent Phosphatidylethanolamin, etwa 2 bis etwa 5 Gewichtsprozent Sphingomyelin, etwa 2 bis etwa 12 Gewichtsprozent Phosphatidylserin, etwa 1 bis etwa 5 Gewichtsprozent Phosphatidylinositol, und etwa 0,2 bis etwa 1 Gewichtsprozent Gangliosid, oder
(j) etwa 20 bis etwa 40 Gewichtsprozent Lipid, etwa 5 bis etwa 30 Gewichtsprozent Phospholipide, etwa 1 bis etwa 5 Gewichtsprozent Phosphatidylcholin, etwa 2 bis etwa 8 Gewichtsprozent Phosphatidylethanolamin, etwa 0,5 bis etwa 5 Gewichtsprozent Sphingomyelin, etwa 1 bis etwa 10 Gewichtsprozent Phosphatidylserin, etwa 1 bis etwa 6 Gewichtsprozent Phosphatidylinositol, und etwa 0,8 bis etwa 3,5 Gewichtsprozent Gangliosid.

14. Mittel zur Verwendung nach einem der Ansprüche 1 bis 13, wobei das Mittel ferner Lactoferrin, Eisen-Lactoferrin, ein Lactoferrinfragment, oder ein Eisen-Lactoferrinfragment, oder eine Kombination von zweien oder mehreren davon umfasst.

15. Verwendung des Mittels nach einem der vorhergehenden Ansprüche, wobei das Mittel (a) eine oder mehrere Kuhmilchfettzusammensetzungen umfasst, die etwa 5 bis etwa 95 Gewichtsprozent Lipid, etwa 0 bis etwa 75 Gewichtsprozent Protein, etwa 5 bis etwa 85 Gewichtsprozent Phospholipid und etwa 0 bis 5 Gewichtsprozent Gangliosid ausgewählt aus
i. einer oder mehreren phospholipidangereicherten Milchfettfraktionen,
ii.einer oder mehreren gangliosidangereicherten Milchfettfraktionen,
iii. einem oder mehreren Hydrolysaten von einem oder mehreren von (i) bis (ii), und
einer Kombination aus zwei oder mehreren von (i) bis (iii) umfassen, zur Herstellung eines Arzneimittels zur Verwendung bei der Behandlung oder Verhinderung von Rotavirusinfektionen in einem Individuum, wobei das Rotavirus ein Neuraminidase-resistentes Rotavirus ist.

## Revendications

1. Agent, comprenant
(a) une ou plusieurs compositions de matière grasse de lait de vache, comprenant d'environ 5 à environ 95% p/p de lipides, d'environ 0 à environ 75% p/p de protéines, d'environ 5 à environ 85% p/p de phospholipides et d'environ 0 à environ 5% p/p de gangliosides, choisies parmi
(i) une ou plusieurs fractions de matière grasse du lait enrichies en phospholipides,
(ii) une ou plusieurs fractions de matière grasse du lait enrichies en gangliosides,
(iii) un ou plusieurs hydrolysats de l'une ou plusieurs quelconques parmi (i) à (ii), et
(iv) une combinaison de deux, ou plus, quelconques, parmi (i) à (iii) ;
pour une utilisation dans le traitement ou la prévention d'une infection par rotavirus chez un sujet, où le rotavirus est un rotavirus résistant à la neuraminidase.

2. Agent pour une utilisation selon la revendication 1, où le rotavirus est un rotavirus humain.

3. Agent pour une utilisation selon la revendication 1 ou 2, où le sujet est un humain, un enfant, un nourrisson, un enfant immuno-vulnérable, un nourrisson immuno-vulnérable, un adulte, un adulte âgé de plus de 55 ans, un adulte immuno-vulnérable, ou un adulte immuno-vulnérable âgé de plus de 55 ans.

4. Agent pour une utilisation selon l'une quelconque des revendications 1 à 3, pour le traitement ou la prévention d'une infection par rotavirus diarrhéogène ou pour le traitement ou la prévention d'une diarrhée provoquée par une infection par rotavirus.

5. Agent pour une utilisation selon l'une quelconque des revendications 1 à 4, où l'agent comprend en outre de la matière grasse de lait anhydre (AMF) comprenant d'environ 40 à environ 100% de lipides.

6. Agent pour une utilisation selon l'une quelconque des revendications 1 à 5, comprenant une ou plusieurs fractions de matière grasse du lait de vache enrichies en phospholipides, ou une ou plusieurs fractions de matière grasse du lait de vache enrichies en gangliosides, ou une combinaison de celles-ci, pour le traitement ou la prévention d'une infection par rotavirus chez un sujet.

7. Agent pour une utilisation selon l'une quelconque des revendications 1 à 6, où la fraction enrichie en phospholipides est choisie parmi le babeurre, une ou plusieurs fractions de babeurre, le sérum de beurre, une ou plusieurs fractions de sérum de beurre, le bêta-sérum, une ou plusieurs fractions de bêta-sérum, une ou plusieurs fractions de sphingolipides, une ou plusieurs fractions de lipides membranaires des globules gras du lait, une ou plusieurs fractions de phospholipides, une ou plusieurs fractions de lipides complexes, et une combinaison quelconque de deux, ou plus, quelconques parmi ceux-ci, où le bêta-sérum est séparé d'un courant laitier contenant plus de 60% de matière grasse, le courant laitier étant constitué de crème ayant subi une inversion de phase passant d'une émulsion huile-dans-eau à eau-dans-huile lors de la production d'huile de beurre ou de matière grasse anhydre de lait à partir de crème.

8. Agent pour une utilisation selon l'une quelconque des revendications 1 à 6, où la fraction enrichie en gangliosides est choisie parmi le babeurre, une ou plusieurs fractions de babeurre, le sérum de beurre, une ou plusieurs fractions de sérum de beurre, le bêta-sérum, une ou plusieurs fractions de bêta-sérum, une ou plusieurs fractions de bêta-sérum enrichies en GD-3, une ou plusieurs fractions de bêta-sérum enrichies en GM-3, une ou plusieurs fractions de bêta-sérum enrichies en GD-3 et GM-3, et une combinaison quelconque de deux, ou plus, quelconques parmi ceux-ci, où le bêta-sérum est séparé d'un courant laitier contenant plus de 60% de matière grasse, le courant laitier étant constitué de crème ayant subi une inversion de phase passant d'une émulsion huile-dans-eau à eau-dans-huile lors de la production d'huile de beurre ou de matière grasse anhydre de lait à partir de crème.

9. Agent pour une utilisation selon l'une quelconque des revendications 1 à 4, comprenant en outre de la matière grasse de lait de vache anhydre (AMF), une ou plusieurs fractions d'AMF, ou une combinaison de celles-ci, pour le traitement ou la prévention d'une infection par rotavirus chez un sujet.

10. Agent pour une utilisation selon l'une quelconque des revendications 1 à 4, où l'agent comprend en outre une fraction d'AMF choisies parmi une ou plusieurs fractions de matière grasse de lait dure, une ou plusieurs fractions de matière grasse de lait molle, une combinaison de fractions de matière grasse de lait dure, une combinaison de fractions de matière grasse de lait molle, une combinaison de fractions de matière grasse de lait dure et de fractions de matière grasse de lait molle, et une combinaison quelconque de deux, ou plus, quelconques parmi celles-ci.

11. Agent pour une utilisation selon la revendication 10, où l'AMF comprend d'environ 98 à environ 100% de matière grasse de lait.

12. Agent pour une utilisation selon l'une quelconque des revendications 6 à 8, où la fraction comprend
(a) d'environ 15 à environ 95% p/p de lipides et d'environ 0 à environ 75% p/p de protéines, ou
(b) d'environ 15 à environ 95% p/p de lipides, d'environ 0 à environ 65% p/p de protéines, d'environ 5 à environ 70% p/p de phospholipides et d'environ 0 à environ 2,5% p/p de gangliosides.

13. Agent pour une utilisation selon l'une quelconque des revendications 6 à 8, où la fraction comprend
(a) d'environ 25 à environ 35% p/p de protéines, d'environ 12 à environ 25% p/p de lipides, d'environ 5 à environ 15% p/p de phospholipides, d'environ 5 à environ 15% p/p de protéines MFGM, et d'environ 0,2 à environ 0,9% p/p de gangliosides, ou
(b) d'environ 40 à environ 60% p/p de protéines, d'environ 25 à environ 45% p/p de lipides, d'environ 10 à environ 25% p/p de phospholipides, d'environ 5 à environ 20% p/p de protéines MFGM, et d'environ 0,5 à environ 2,0% p/p de gangliosides, ou
(c) d'environ 50 à environ 70% p/p de protéines, d'environ 12 à environ 32% p/p de lipides, d'environ 5 à environ 25% p/p de phospholipides, d'environ 2 à environ 8% p/p de phosphatidylcholine, d'environ 2 à environ 10% p/p de phosphatidyléthanolamine, d'environ 2 à environ 8% p/p de sphingomyéline, et d'environ 1 à environ 3% p/p de phosphatidylsérine, d'environ 10 à environ 20% p/p de protéines MFGM, et d'environ 0,5 à environ 2,5% p/p de gangliosides, ou
(d) d'environ 0 à environ 10% p/p de protéines, d'environ 85 à environ 97% p/p de lipides, d'environ 25 à environ 35% p/p de phospholipides, d'environ 5 à environ 10% p/p de phosphatidylcholine, d'environ 7 à environ 13% p/p de phosphatidyléthanolamine, d'environ 4 à environ 9% p/p de sphingomyéline, d'environ 2 à environ 5% p/p de phosphatidylsérine, d'environ 1 à environ 3% p/p de phosphatidylinositol, d'environ 0 à environ 5% p/p de protéines MFGM, et d'environ 1 à environ 3% p/p de gangliosides, ou
(e) d'environ 10 à environ 15% p/p de protéines, d'environ 80 à environ 95% p/p de lipides, d'environ 60 à environ 80% p/p de phospholipides, d'environ 10 à environ 20% p/p de phosphatidylcholine, d'environ 18 à environ 28% p/p de phosphatidyléthanolamine, d'environ 10 à environ 20% p/p de sphingomyéline, d'environ 4 à environ 12% p/p de phosphatidylsérine, d'environ 2 à environ 10% p/p de phosphatidylinositol, d'environ 0 à environ 5% p/p de protéines MFGM, et d'environ 1 à environ 5% p/p de gangliosides, ou
(f) d'environ 75 à environ 99% p/p de lipides, d'environ 15 à environ 35% p/p de phospholipides, d'environ 5 à environ 15% p/p de phosphatidylcholine, d'environ 5 à environ 15% p/p de phosphatidyléthanolamine, d'environ 4 à environ 15% p/p de sphingomyéline, d'environ 0,1 à environ 2% p/p de phosphatidylsérine, et d'environ 0,1 à environ 2% p/p de phosphatidylinositol, ou
(g) d'environ 75 à environ 95% p/p de lipides, d'environ 50 à environ 90% p/p de phospholipides, d'environ 10 à environ 45% p/p de phosphatidylcholine, d'environ 12 à environ 25% p/p de phosphatidyléthanolamine, d'environ 12 à environ 25% p/p de sphingomyéline, d'environ 1 à environ 6% p/p de phosphatidylsérine, et d'environ 0,5 à 4% p/p de phosphatidylinositol, ou
(h) d'environ 80 à environ 90% p/p de lipides, d'environ 65 à environ 75% p/p de phospholipides, d'environ 10 à environ 30% p/p de phosphatidylcholine, d'environ 12 à environ 22% p/p de phosphatidyléthanolamine, d'environ 12 à environ 22% p/p de sphingomyéline, et d'environ 1 à environ 3% p/p de phosphatidylsérine, ou
(i) d'environ 25 à environ 45% p/p de lipides, d'environ 10 à environ 30% p/p de phospholipides, d'environ 2 à environ 5% p/p de phosphatidylcholine, d'environ 3 à environ 7% p/p de phosphatidyléthanolamine, d'environ 2 à environ 5% p/p de sphingomyéline, d'environ 2 à environ 12% p/p de phosphatidylsérine, d'environ 1 à environ 5% p/p de phosphatidylinositol, et d'environ 0,2 à environ 1% p/p de gangliosides, ou
(j) d'environ 20 à environ 40% p/p de lipides, d'environ 5 à environ 30% p/p de phospholipides, d'environ 1 à environ 5% p/p de phosphatidylcholine, d'environ 2 à environ 8% p/p de phosphatidyléthanolamine, d'environ 0,5 à environ 5% p/p de sphingomyéline, d'environ 1 à environ 10% p/p de phosphatidylsérine, d'environ 1 à environ 6% p/p de phosphatidylinositol, et d'environ 0,8 à environ 3,5% p/p de gangliosides.

14. Agent pour une utilisation selon l'une quelconque des revendications 1 à 13, où l'agent comprend en outre de la lactoferrine, de la lactoferrine-fer, un fragment de lactoferrine, ou un fragment de lactoferrine-fer, ou une combinaison de deux, ou plus, quelconques parmi celles-ci.

15. Utilisation de l'agent tel qu'exposé selon l'une quelconque des revendications précédentes, l'agent comprenant
(a) une ou plusieurs compositions de matière grasse de lait de vache, comprenant d'environ 5 à environ 95% p/p de lipides, d'environ 0 à environ 75% p/p de protéines, d'environ 5 à environ 85% p/p de phospholipides et d'environ 0 à environ 5% p/p de gangliosides, choisies parmi
(i) une ou plusieurs fractions de matière grasse du lait enrichies en phospholipides,
(ii) une ou plusieurs fractions de matière grasse du lait enrichies en gangliosides,
(iii) un ou plusieurs hydrolysats de l'une ou plusieurs quelconques parmi (i) à (ii), et
une combinaison de deux, ou plus, quelconques, parmi (i) à (iii) ; pour l'élaboration d'un médicament destiné à une utilisation dans le traitement ou la prévention d'une infection par rotavirus chez un sujet, où le rotavirus est un rotavirus résistant à la neuraminidase.
